(19) Europäisches Patentamt
European Patent Office
Office européen des brevets

(11) EP 1 568 680 A1

# (12) EUROPEAN PATENT APPLICATION

(43) Date of publication:
31.08.2005 Bulletin 2005/35

(51) Int Cl.[7]: **C07C 69/738**, B41M 5/38

(21) Application number: 05101218.5

(22) Date of filing: 18.02.2005

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: 27.02.2004 JP 2004053357

(71) Applicant: **Konica Minolta Photo Imaging, Inc.**
**Shinjuku-ku, Tokyo 163-0512 (JP)**

(72) Inventors:
- **Ikemizu, Dai**
  **Hachioji-shi 192-8505, Tokyo (JP)**
- **Suzuki, Takatugu**
  **Hachioji-shi 192-8505, Tokyo (JP)**
- **Ikesu, Satoru**
  **Hachioji-shi 192-8505, Tokyo (JP)**

(74) Representative: **Gille Struck Neidlein Prop Roos**
**Patentanwälte**
**Brucknerstrasse 20**
**40593 Düsseldorf (DE)**

## (54) Thermal transfer recording material, thermal transfer image receptive sheet, ink sheet, thermal transfer recording method, and metal containing compound

(57) A thermal transfer recording material including a support having thereon a metal containing compound derived from a divalent metal salt and a compound represented by Formula (I), the metal containing compound having a property to form a metal-chelated dye by heating with a metal chelating dye represented by Formulas (1), (2) or (4).

EP 1 568 680 A1

## Description

### FIELD OF THE INVENTION

The present invention relates to a thermal transfer recording material, a thermal transfer image receptive sheet, an ink sheet, a thermal transfer recording method, and a metal containing compound.

### BACKGROUND OF THE INVENTION

Heretofore, as a technique to form chromatic or monochromatic images, known has been sublimation transfer which utilizes diffusible sublimation dyes during heating. Whereby it is possible to digitally output dry full color images of high quality, which are comparable to silver halide photography. In this transfer method, an ink sheet containing sublimation dyes is placed face to face with the image receptive layer of an image receptive sheet, and subsequently, images are formed by imagewise transferring sublimation dyes onto the image receptive layer employing a heating device such as a thermal head or laser means. In such thermal transfer recording, sublimation dyes employed in the ink sheet play an important role. However, since conventional sublimation dyes are subjected to diffusion transfer employing heat, they are very sensitive to heat and after transferring images onto an image receptive layer, diffusion continues, causing problems in which images are subjected to bleeding. Further, problems result in which the storage stability of images such as dark fading or light fading is insufficient.

Consequently, in order to overcome these drawbacks, an image forming method (a reactive sublimation transfer method) is proposed in which images are formed by allowing dye precursors to react with a dye fixing body or employing so-called reactive type dyes. For example, a method (refer, for example, to Patent Documents 1 - 3) is disclosed in which employed as dye precursors are chelatable heat fusible compounds (hereinafter also referred to as metal-chelating dyes or post-chelate dyes) which when allowed to react with metal ion-containing compounds (hereinafter also referred to as metal sources) which are dye fixing bodies to form metal chelates, images are formed. According to this method, image bleeding is reduced compared to general sublimation transfer images. However, when stored at high temperature and high humidity conditions, bleeding and in addition, non-image transfer portions (being white backgrounds) are subject to yellowing and the resulting quality is still inferior to common printed images.

In order to improve image retention properties such as dark fading and light fading, it is considered to make dyes themselves more resistant to effects of light, oxygen, and moisture, but the resulting resistance is limited. In the reactive sublimation transfer method, since the post-chelate dyes are transferred on the image receptive layer surface side and subsequently diffused, formed dyes tend to be localized near the surface, making difficult to achieve uniform distribution in the image receptive layer. As a result, dyes near the surface tend to be directly affected by light, oxygen and moisture, resulting in problems in which fading is accelerated. On the other hand, a technique is reported in which in the above reactive sublimation transfer method, a protective layer is provided on the surface of the image receptive layer employing a heating device. This technique exhibits less mechanical abrasion. However, problems occurred in which when dyes were localized on the surface of an ink receptive layer, dyes diffused into the protective layer and image durability was degraded. In the same manner, when post-chelate dyes are successively transferred from an ink sheet to the image receptive layer, a so-called "reverse transfer" problem occurs in which dyes are localized in the image receptive layer surface due to the above reason, and when the subsequent ink layer is transferred, the previous dyes are somewhat adsorbed. Consequently, it has been difficult to achieve recording at high photographic speed and high density.

Further, colorants, known as dyes and pigments, are widely employed in various forms such as fiber dying materials, resin and paint coloring materials, image forming materials in photography, printing, copiers, and printers, or light absorbing materials in color filters. In recent years, various image forming dyes are proposed for colored hard copy, employing electrophotography, silver salt photography, and thermal transfer. Along with the progress of electronic imaging, demand for filter dyes for solid state image sensing tubes and color liquid crystal television as well as dyes for light recording media utilizing semiconductor lasers has increased and dye using fields have expanded. The following properties are commonly desired for all the above dyes; namely, exhibiting preferable hue in terms of color reproduction, optimal spectral absorption characteristics, and desired image durability such as heat resistance, moisture resistance, or chemical resistance, as well as a high molar absorption coefficient. Heretofore, azo dyes have been employed as yellow, magenta and cyan thermographic, photothermographic or thermal transfer recording materials; azomethine dyes have been employed as image forming dyes of silver halide color photographic materials based on a subtractive color system utilizing yellow, magenta, and cyan; while methine dyes have been employed as filter dyes and anti-halation dyes of silver halide photographic materials. These dyes are disclosed in main publications (refer, for example, to Patent Documents 4 - 9), and are proposed as image forming dyes for colored hard copy. However, these dyes have not provided sufficient image durability. Further, metal complex dyes which exhibit high light-fastness are described (refer, for example, to Patent Documents 10 - 13). However, these dyes are not desirable for image

formation due to the fact that the molar absorption coefficient is relatively low and the absorption wavelengths are very specific. Consequently, a development of dyes provided with the above properties is demanded.

(Patent Document 1) Japanese Patent Publication Open to Public Inspection (hereinafter referred to as JP-A) No. 59-78893
(Patent Document 2) JP-A No. 59-109394
(Patent Document 3) JP-A No. 60-2398
(Patent Document 4) JP-A No. 59-184339
(Patent Document 5) JP-A No. 60-130735
(Patent Document 6) JP-A No. 2-3450
(Patent Document 7) JP-A No. 4-78583
(Patent Document 8) JP-A No. 4-89287
(Patent Document 9) JP-A No. 4-359968
(Patent Document 10) JP-A No. 64-44786
(Patent Document 11) JP-A No. 2-76884
(Patent Document 12) JP-A No. 5-17701 (claims)
(Patent Document 13) JP-A No. 9-143382 (claims)

## SUMMARY OF THE INVENTION

In view of the foregoing, the present invention was achieved. One object of the present invention is to provide a thermal transfer recording material which exhibits high photographic speed and excellent image lasting quality, such as excellent light-fastness, almost no image bleeding under high temperature and high humidity, or no yellow staining on the white background, the image receptive layer, as well as a thermal transfer image forming method which results in high photographic speed and makes it possible to prepare images exhibiting excellent image lasting quality. Another object of the present invention is to provide a thermal transfer recording sheet which exhibits high photographic speed and contains chelating dyes exhibiting excellent light, heat and humidity fastness after forming chelated-dyes, and a transfer method using the same sheet.

An aspect of the present invention is a thermal transfer recording material comprising a support having thereon a metal containing compound derived from a divalent metal salt and a compound represented by Formula (I).

Another aspect of the present invention is an ink sheet comprising a support having thereon an ink layer containing:

(a) a metal-containing compound derived from a divalent metal salt and a compound represented by Formula (I); and
(b) a metal-chelating dye represented by Formulas (1), (2) or (4).

Another aspect of the present invention is a metal containing compound derived from a divalent metal salt and a compound represented by Formula (I).

Formulas (I), (1), (2) and (4) will be described below.

## BRIEF DESCRIPTION OF THE DRAWINGS

Fig. 1(a) and Fig. 1(b) are top views each showing one example of an ink layer and a re-heating layer provided on the ink sheet employed in the method of the present invention.
Fig. 2(a), Fig. 2(b) and Fig. 2(c) are schematic views each showing one example of a thermal transfer recording apparatus employed in the method of the present invention.

## DESCRIPTION OF THE PREFERRED EMBODIMENTS

It is possible to achieve the above objects of the present invention employing the embodiments below.

(1) A thermal transfer recording material comprising a support having thereon a metal containing compound derived from a divalent metal salt and a compound represented by Formula (I):

Formula (I)

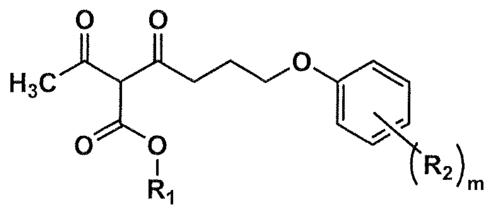

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provide that when m is 2 or more, a plurality of $R_2$s may be the same or different.

(2) The thermal transfer recording material described in the aforesaid item 1,

wherein $R_1$ in Formula (I) is an alkyl group having two carbon atoms or more.

(3) The thermal transfer recording material described in the aforesaid items 1 or 2,

wherein a hydrophilicity (a logP value) of the compound represented by Formula (I) is between 4.0 and 10.0.

(4) A thermal transfer image receptive sheet comprising a support having thereon an image receptive layer containing the metal containing compound described in any one of the aforesaid items 1 - 3.

(5) A method of forming a metal-chelated dye image comprising the steps of:

(a) superimposing an ink sheet comprising a support having thereon an ink layer containing a metal-chelating dye onto the thermal transfer image receptive sheet of the above-described item 4 so that a surface of the ink layer of the ink sheet faces a surface of the image receptive layer of the thermal transfer image receptive sheet; and

(b) imagewise heating the ink sheet and the thermal transfer image receptive sheet superimposed so as to form a metal-chelated dye by a reaction of the metal-chelating dye and the metal containing compound in the thermal transfer image receptive sheet described in the aforesaid item 4.

(6) An ink sheet comprising a support having thereon an ink layer containing a metal-chelated dye derived from:

(a) a metal-containing compound derived from a divalent metal salt and a compound represented by Formula (I):

Formula (I)

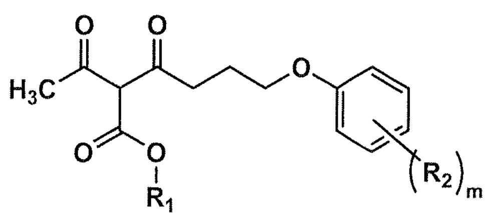

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provide that when m is 2 or more, a plurality of $R_2$s may be the same or different; and

(b) a metal-chelating dye represented by Formulas (1), (2) or (4):

Formula (1)

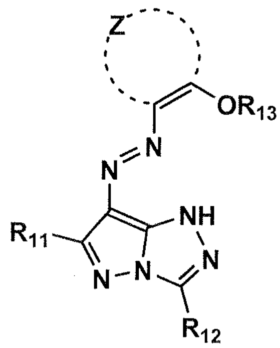

wherein $R_{11}$ and $R_{12}$ each represent a hydrogen atom or a substituent; $R_{13}$ represents an alkyl or an aryl group which may have a substituent; and Z represents a group of atoms necessary to form a 5- to 6-membered aromatic ring together with two carbon atoms;

Formula (2)

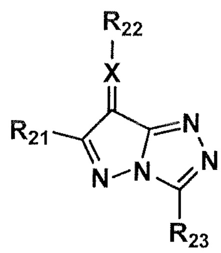

wherein $R_{21}$ represents a hydrogen atom, a halogen atom, or a univalent substituent; $R_{22}$ represents an aromatic carbon ring group or an aromatic heterocyclic group; X represents a methine group or a nitrogen atom; $R_{23}$ represents Formula (3), below; Y represents a group of atoms necessary to form a hetero-aromatic ring; and $X_1$ represents a carbon atom or a nitrogen atom;

Formula (3)

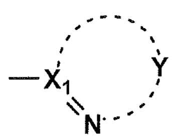

Formula (4)

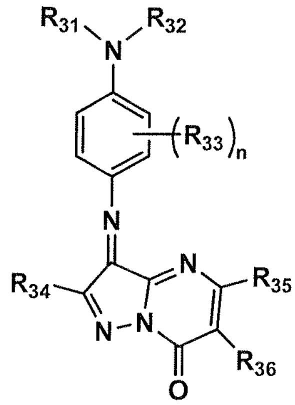

wherein $R_{31}$ and $R_{32}$ each represent a substituted or unsubstituted aliphatic group; $R_{33}$ represents a substituent; n represents an integer of 0 - 4, provided that when n is 2 or more, a plurality of $R_{33}$ may be the same or different; $R_{34}$, $R_{35}$, and $R_{36}$ each represent an alkyl group in which they may be the same or different, however, $R_{35}$ and $R_{36}$ each represent an alkyl group having 3 - 8 carbon atoms.

(7) A method of forming a metal-chelated dye image comprising the steps of:

(a) superimposing the ink sheet described in the aforesaid item 6 onto a thermal transfer image receptive sheet comprising a support having thereon an image receptive layer including a metal containing compound so that a surface of the ink layer of the ink sheet faces a surface of the image receptive layer of the thermal transfer image receptive sheet; and
(b) imagewise heating the ink sheet and the thermal transfer image receptive sheet superimposed so as to form a metal-chelated dye by a reaction of the metal-chelating dye and the metal containing compound in the thermal transfer image receptive sheet.

(8) A metal containing compound derived from a divalent metal salt and a compound represented by Formula (I):

Formula (I)

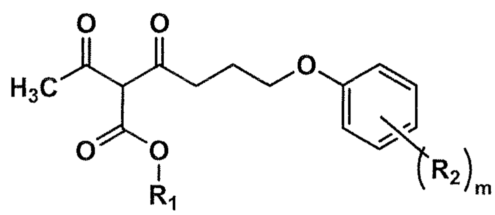

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provide that when m is 2 or more, a plurality of $R_2$s may be the same or different.

Based on the present invention, it is possible to provide a thermal transfer recording material which exhibits high photographic speed and excellent image lasting quality such as excellent light-fastness, almost no image bleeding under high temperature and high humidity, or no yellow staining on white backgrounds, an image receptive layer, and a thermal transfer image forming method which results in high photographic speed and makes it possible to prepare images exhibiting excellent image lasting quality, and further to provide a thermal transfer recording sheet which exhibits high photographic speed and in which chelating dyes are employed which exhibit excellent light, heat and humidity fastness, and a thermal transfer method.

The present invention will now be further detailed. In aforesaid Formula (I), $R_1$ represents an alkyl group having at least two carbon atoms (e.g., an ethyl group, a propyl group, a butyl group, an octyl group, an ethoxyethyl group, an isopropyl group, a t-butyl group, and a 2-ethylhexyl group); a cycloalkyl group (e.g., a cyclohexyl group and

a cycloheptyl group); an alkenyl group (e.g., an ethenyl group, an allyl group, and a butenyl group); an alkynyl group (e.g., an ethynyl group, a propynyl group, and a butynyl group); an aryl group (e.g., a phenyl group, a tolyl group, a chlorophenyl group, and a biphenyl group); an aralkyl group (e.g., a benzyl group and an o-bromobenzyl group); a heterocyclic group (e.g., a pyrrolyl group, a thienyl group, an imidazolyl group, a furanyl group, a pyridinyl group, a benzothiazolyl group, a thiadiazolyl group, and a benzothiazolyl group). These substituents may further have a substituent and may be substituted with a halogen atom (e.g., a fluorine atom, a chlorine atom, and a bromine atom); an alkyl group (e.g., a methyl group, an ethyl group, a propyl group, a butyl group, a pentyl group, an iso-pentyl group, a 2-ethyl-hexyl group, an octyl group, and a decyl group); a cycloalkyl group (e.g., a cyclohexyl group and a cycloheptyl group); an alkenyl group (e.g., an ethenyl-2-propenyl group, a 3-butenyl group, a 1-methyl-3-propenyl group, a 3-pentenyl group, and a 1-methyl-3-butenyl group); a cycloalkenyl group (e.g., a 1-cycloalkenyl group and a 2-cycloalkenyl group); an alkynyl group (e.g., an ethynyl group and a 1-propynyl group); an alkoxy group (e.g., a methoxy group, an ethoxy group, and a propoxy group); an alkylcarbonyloxy group (e.g., an acetyloxy group); an alkylthio group (e.g., a methyloxy group and a trifluoromethylthio group); a carboxyl group; an alkylcarbonylamino group (e.g., an acetyl amino group); a ureido group (e.g., a methylaminocarbonylamino group); an alkylsulfonylamino group (e.g., a methanesulfonylamino group); an alkylsulfonyl group (e.g., a methanesulfonyl group and a trifluoromethanesulfonyl group); a carbamoyl group (e.g., a carbamoyl group, an N,N-dimethylcarbamoyl group, and an N-morpholinocarbonyl group); a sulfamoyl group (e.g., a sulfamoyl group, an N,N-dimethylsulfamoyl group, and a morpholinosulfamoyl group); a trifluoromethyl group, a hydroxy group, a nitro group, and a cyano group; an alkylsulfoneamido group (e.g., a methanesulfonamido group and a butanesulfonamido groups); an alkylamino group (e.g., an amino group, an N,N-diethylamino group, an N,N-diethylamino group); a sulfo group, a sulfono group, a sulfite group, a sulfino group; an alkylsulfonylaminocarbonyl group (e.g., a methanesulfonylaminocarbonyl group and an ethanesulfonylaminocarbonyl group); an alkylcarbonylaminosulfonyl group (an acetoamidosulfonyl group and a methoxyacetoamidosulfonyl group); an alkynylaminocarbonyl group (e.g., an acetoamidocarbonyl group and a methoxyacetoamidocarbonyl group); an alkylsulfinylaminocarbonyl group (e.g., a methanesulfinylaminocarbonyl group and an ethanesulfinylaminocarbonyl group); an aryl group (e.g., a phenyl group and a carboxyphenyl group); an aryloxy group (e.g., a phenoxy group, a p-chlorophenoxy group, and a p-tert-butylphenoxy group); a heterocyclic group (a pyrrolyl group, a thienyl group, an imidazolyl group, a furanyl group, a pyridinyl group, a benzothiazolyl group, a thiadiazolyl group, and a benzothiazolyl group). In the case of the presence of at least two substituents, they may be the same or different.

$R_2$ represents a branched alkyl group (for example, an isopropyl group, a butyl group, an isobutyl group, a 2-ethylhexyl group, a t-amyl group, and a t-octyl group).

"m" represents an integer of 1 - 5. Specific examples of the compounds represented by Formula (I) are listed below. However, the present invention is not limited thereto.

I-1

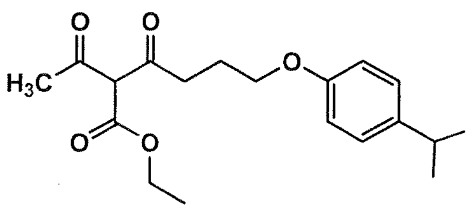

I-2

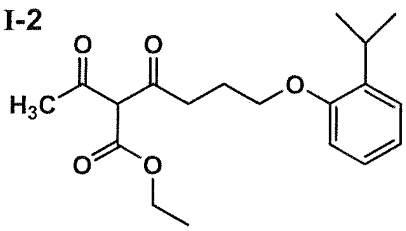

I-3

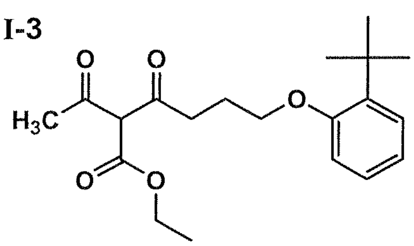

I-4

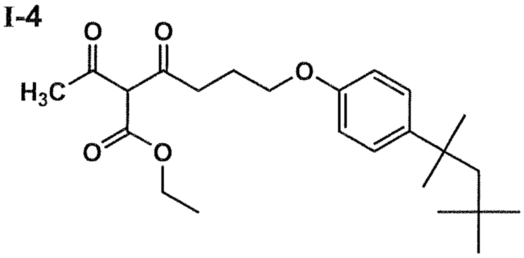

**I-5**

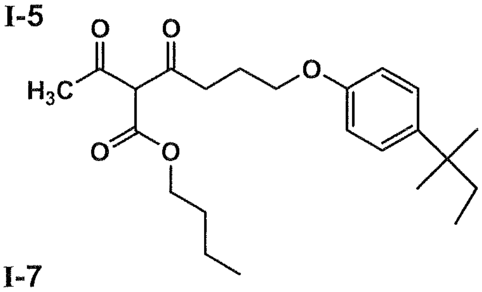

**I-6**

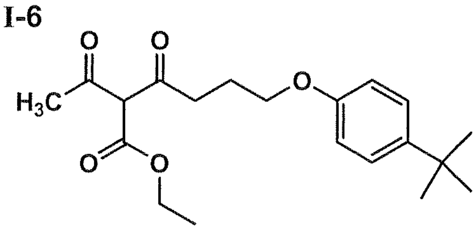

**I-7**

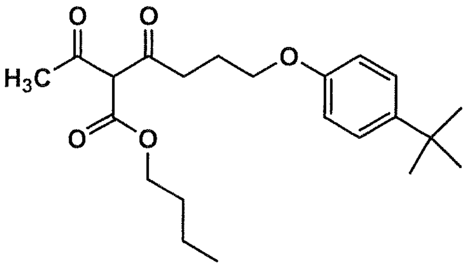

**I-8**

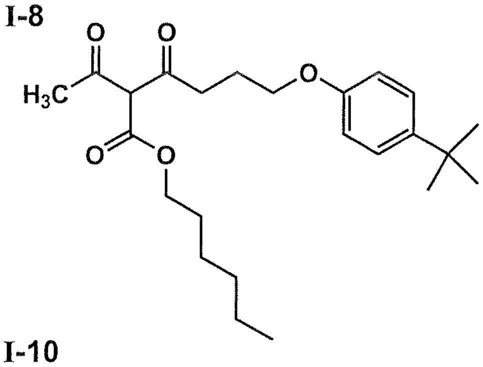

**I-9**

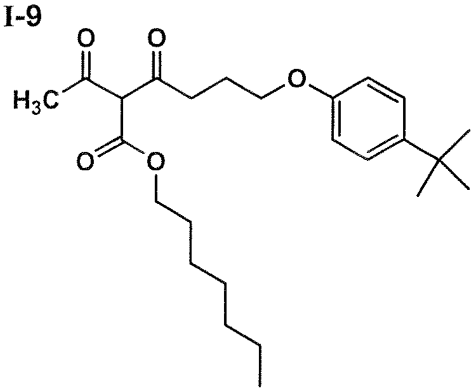

**I-10**

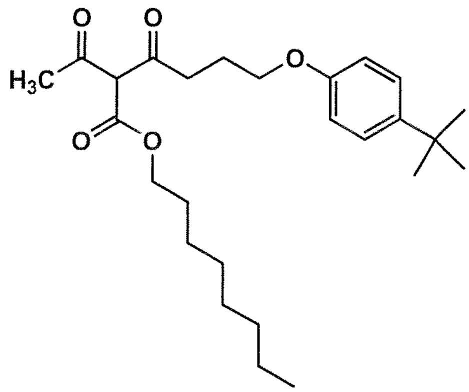

**I-11**

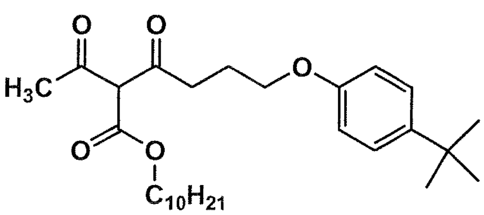

**I-12**

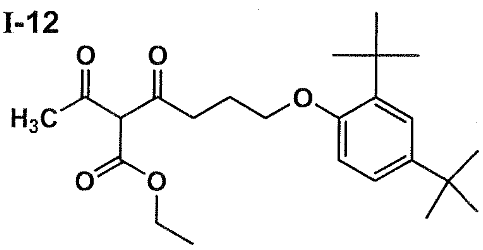

**I-13**

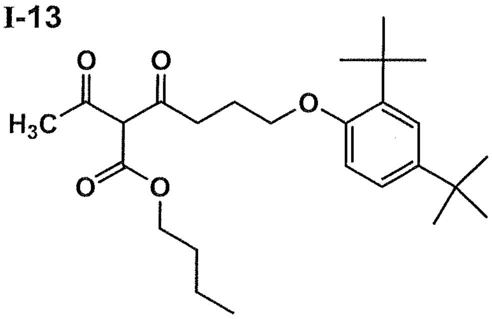

**I-14**

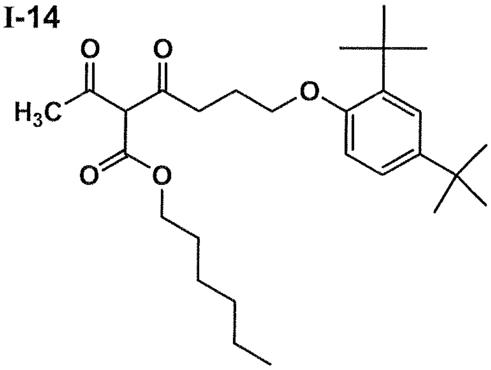

**I-15**

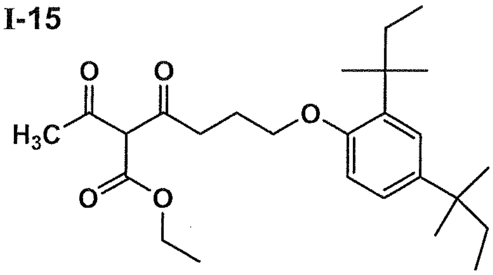

**I-16**

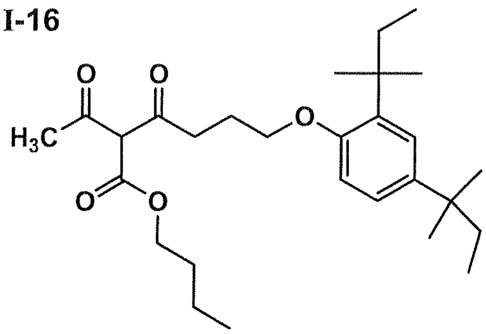

**I-17**

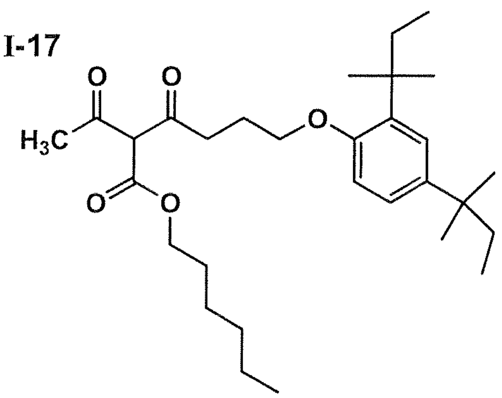

**I-18**

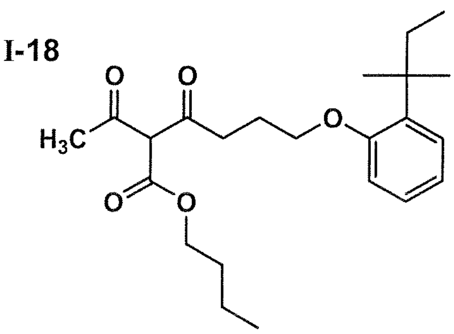

**I-19**

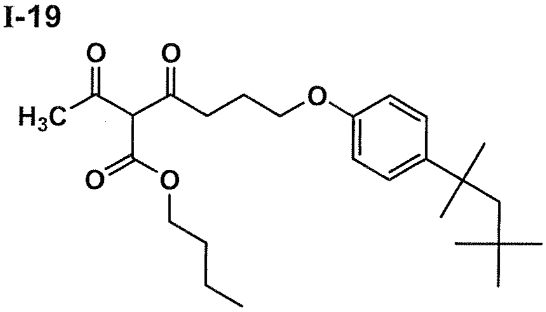

**I-20**

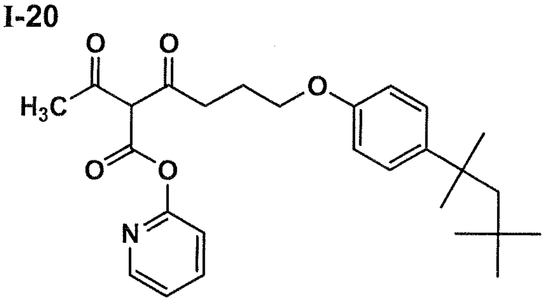

I-21

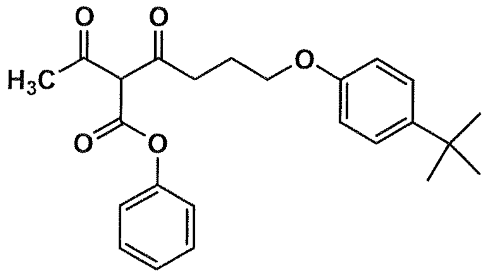

I-22

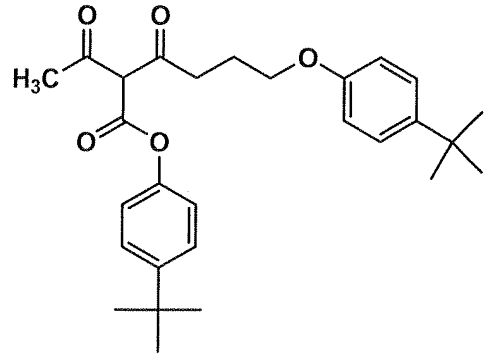

I-23

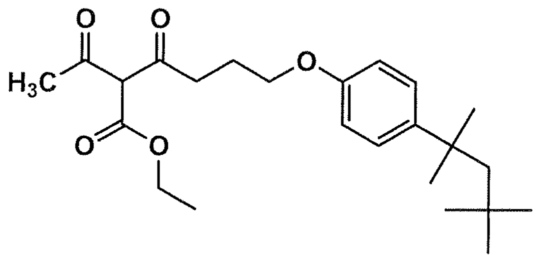

I-24

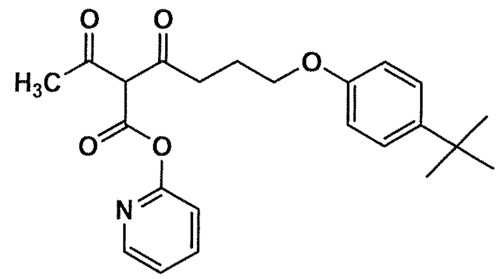

I-25

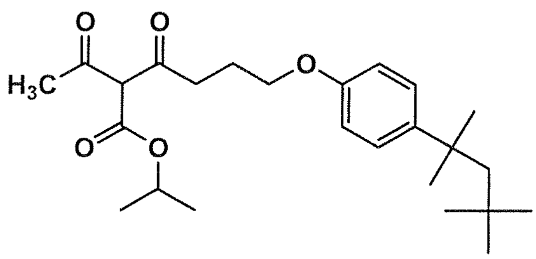

I-26

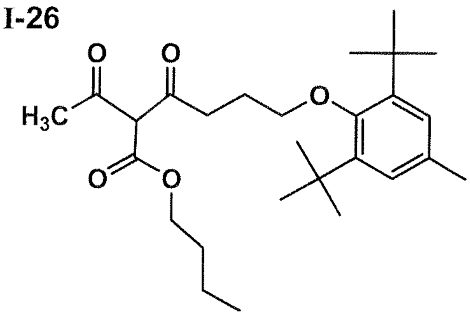

I-27

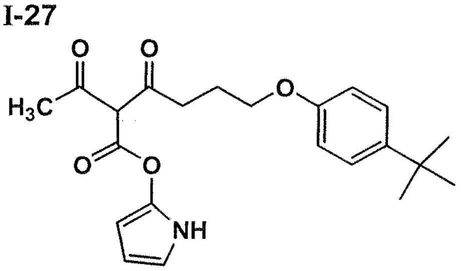

I-28

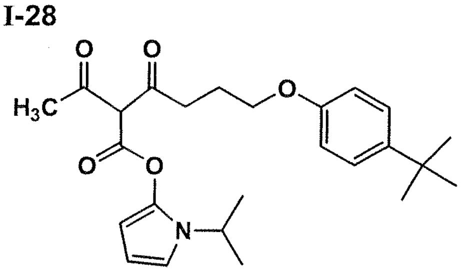

I-29

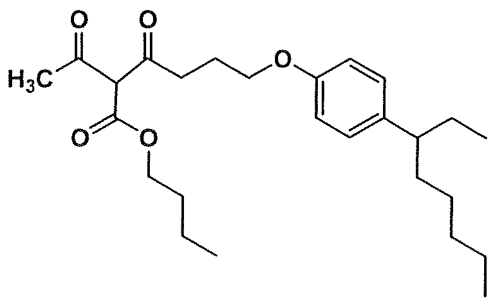

I-30

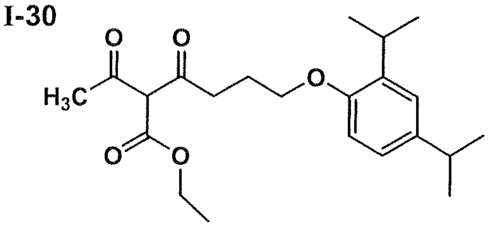

I-31

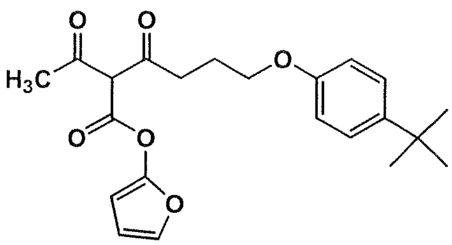

I-32

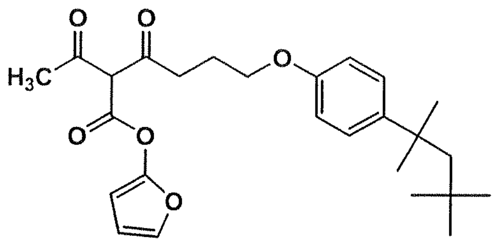

I-33

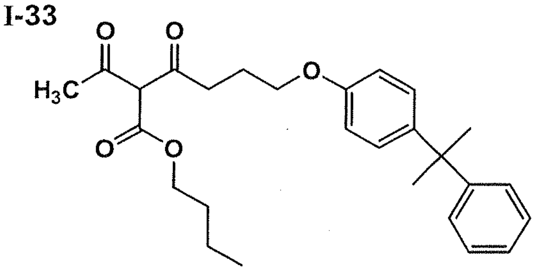

I-34

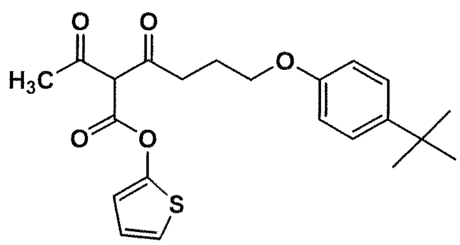

I-35

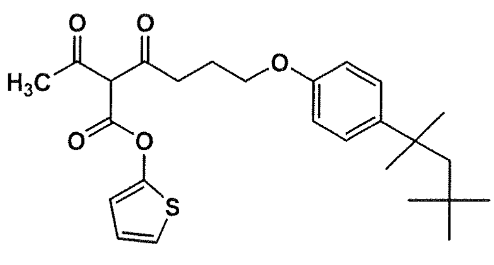

The hydrophilic/hydrophobic parameter value (being the logP value) of the compounds represented by Formula (I) is preferably in the range of 4.0 - 10.0, but is more preferably in the range of 5.0 - 8.0, so that metal containing compounds prepared employing the above compounds exhibit desired performance.

It is possible to commonly determine a hydrophilicity/hydrophobicity exhibiting parameter, logP, based on a partition coefficient of a substance in two solvent systems to n-octanol and water. Such determination methods are detailed on pages 73 - 103 of Kagaku Ryoiki Zokan No. 122 "Yakubutsu no Kozo Kassei Sokan (Structural Activity Correlation of Medicines)" (Nankodo). In recent years, a method has been proposed in which logP is determined by computation. Listed as a particularly useful method may be software available from Molecular Design, Limited. As described in the present invention, logP is the computed value employing the above software "CHEMLAB-II Revision

10.02".

Metal containing compounds employed in the present invention will now be described. The metal containing compounds employed in the present invention are represented by Formula (A) below

$$\text{Formula (A): } M^{2+} (X_{11}^{\ -})_2$$

In the above formula, $M^{2+}$ represents a divalent metal ion. Of divalent metal ions, in view of color of the metal containing compounds themselves and tints of chelated dyes, copper and zinc are preferred. $X_{11}$ represents the compounds represented by Formula (I) capable of forming a complex with divalent metal ions. Listed as representative ligands is $H_2O$ or $NH_3$.

It is preferable that metal containing compounds in the present invention are prepared in such a manner that the compound represented by Formula (I) is synthesized and is subsequently allowed to react with divalent metal compounds. It is possible to synthesize these metal containing compounds, employing the method described in "Chelate Kagaku (Chelate Chemistry) (5) Sakutai Kagaku Jikken Ho (Complex Chemistry Experimental Method [I] (Nankodo)". Listed as employable divalent metal compounds are nickel chloride, nickel acetate, magnesium compounds represented by magnesium chloride, calcium compounds represented by calcium chloride, barium chloride, zinc chloride, zinc acetate, titanium chloride (II), iron chloride (II), copper chloride (II), cobalt chloride, manganese chloride (II), lead chloride, lead acetate, mercury chloride, and mercury acetate. As noted above, in view of color of the metal containing compounds themselves and tint of chelated dyes, zinc chloride, zinc acetate, nickel chloride, and nickel acetate are preferred, and of these, nickel acetate is most preferred. Synthesis examples of metal containing compounds will now be described.

Synthesis Example-1

Synthesis Example 1 (Synthesis of Compound I-8)

Added to 45 ml of acetonitrile were 7.9 g (0.04299 mol) of 3-oxo-butyric acid-n-hexyl ester, 9 ml of an aqueous solution of 9.4 of calcium chloride (0.08458 mol), and 12.8 g 80.1269) of triethylamine. The resulting mixture was stirred for one hour, and thereafter, 11.3 g (0.04440 mol) of butyric chloride was dripped over a period of 20 minutes, while maintaining the interior temperature at 30 - 35 °C. After dripping, the resulting mixture was stirred for an additional two hours. Thereafter, the reaction liquid was washed with water, concentrated, and purified employing column chromatography (hexane/ethyl acetate), whereby 13.3 g (at a yield of 78.3 percent) of Compound I-8. Incidentally, the structure was identified employing $^1$H-NMR, as well as mass spectra.

Synthesis Example 2 (Synthesis of Compound I-19)

Compound I-19 of 16.0 g (at a yield of 85.9 percent) was prepared by performing the reaction, post-treatment and purification employing the same formulation as for Synthesis Example 1, except that 3-oxo-butyric acid-n-hexyl ester was replaced with 6.8 g (0.04299 mol) of 3-oxo-butyric acid-n-butyl ester.

Synthesis Example 3 (Synthesis of Compound I-22)

Compound I-22 of 14.6 g (at a yield of 81.6 percent) was prepared by performing the reaction, post-treatment and purification employing the same formulation as for Synthesis Example 1, except that 3-oxo-butyric acid-n-hexyl ester was replaced with 9.3 g (0.03953 mol) of 3-oxo-butyric acid 4-tert-butylphenyl ester.

Synthesis Example 4 (Synthesis of Metal Complex II-8)

Dissolved in 30 ml of methanol was 3.2 g (0.01305 mol) of nickel acetate tetrahydrate, and thereafter, 10.6 g (0.02611 mol) of Compound I-8 was dripped into the resulting solution over a period of 5 minutes. The resulting mixture underwent reaction at room temperature for one hour, and subsequently was cooled by iced water. Deposited crystals were collected via filtration. After washing the resulting crystals with heptane, they were recrystallized employing approximately 120 ml of methanol, whereby 8.6 g (at a yield of 76.2 percent) of Metal Complex II-8 was obtained. The melting point was 67 - 62 °C.

Synthetic Example 5 (Synthesis of Metal Complex II-19)

Compound II-19 of 11.3 g (at a yield of 81.6 percent) was prepared employing the same reaction, post-treatment and purification as for Synthesis Example 4, except that Compound I-8 was replaced with Compound I-19. The melting point was 65 - 67 °C.

Table 1 shows the compounds represented by Formula (I) prepared as above, logP values thereof, and corresponding metal containing compounds (tentatively, designated as Compounds II and III).

Table 1

Molecular Weight of Compounds Represented by Formula (I), logP and Molecular Weight of Corresponding Metal Complexes

| Compound Represented by Formula (I) | Molecular Weight | logP | Ni Complex | Molecular Weight | Cu Complex | Molecular Weight |
|---|---|---|---|---|---|---|
| I-1 | 334.41 | 3.77 | II-1 | 725.49 | III-1 | 730.35 |
| I-2 | 334.41 | 3.37 | II-2 | 725.49 | III-2 | 730.35 |
| I-3 | 348.43 | 4.17 | II-3 | 753.53 | III-3 | 758.39 |
| I-4 | 404.54 | 6.02 | II-4 | 865.75 | III-4 | 870.61 |
| I-5 | 390.51 | 5.75 | II-5 | 837.69 | III-5 | 842.55 |
| I-6 | 348.43 | 4.17 | II-6 | 753.53 | III-6 | 758.39 |
| I-7 | 376.49 | 5.22 | II-7 | 809.65 | III-7 | 814.51 |
| I-8 | 404.54 | 6.28 | II-8 | 865.75 | III-8 | 870.61 |
| I-9 | 418.57 | 6.81 | II-9 | 893.81 | III-9 | 898.67 |
| I-10 | 432.59 | 7.34 | II-10 | 921.85 | III-10 | 926.71 |
| I-11 | 460.65 | 8.40 | II-11 | 977.97 | III-11 | 982.83 |
| I-12 | 404.54 | 5.99 | II-12 | 865.75 | III-12 | 870.61 |
| I-13 | 432.59 | 7.05 | II-13 | 921.85 | III-13 | 926.71 |
| I-14 | 460.65 | 8.11 | II-14 | 977.97 | III-14 | 982.83 |
| I-15 | 432.59 | 7.05 | II-15 | 921.85 | III-15 | 926.71 |
| I-16 | 460.65 | 8.11 | II-16 | 977.97 | III-16 | 982.83 |
| I-17 | 488.70 | 9.17 | II-17 | 1034.07 | III-17 | 1038.93 |
| I-18 | 390.51 | 5.75 | II-18 | 837.69 | III-18 | 842.55 |
| I-19 | 432.59 | 7.08 | II-19 | 921.85 | III-19 | 926.71 |
| I-20 | 453.57 | 5.85 | II-20 | 963.81 | III-20 | 968.67 |
| I-21 | 396.48 | 4.99 | II-21 | 849.63 | III-21 | 854.49 |
| I-22 | 452.58 | 6.81 | II-22 | 961.83 | III-22 | 966.69 |
| I-23 | 404.54 | 6.02 | II-23 | 865.75 | III-23 | 870.61 |
| I-24 | 397.46 | 3.99 | II-24 | 851.59 | III-24 | 856.45 |
| I-25 | 418.57 | 6.33 | II-25 | 893.81 | III-25 | 898.67 |
| I-26 | 446.62 | 7.55 | II-26 | 949.91 | III-26 | 954.77 |
| I-27 | 385.45 | 3.73 | II-27 | 827.57 | III-27 | 832.43 |
| I-28 | 427.53 | 4.87 | II-28 | 911.73 | III-28 | 916.59 |
| I-29 | 432.59 | 7.60 | II-29 | 921.85 | III-29 | 926.71 |
| I-30 | 376.49 | 4.79 | II-30 | 809.65 | III-30 | 814.51 |
| I-31 | 386.44 | 4.16 | II-31 | 829.55 | III-31 | 834.41 |
| I-32 | 442.54 | 6.02 | II-32 | 941.75 | III-32 | 946.61 |
| I-33 | 438.56 | 6.26 | II-33 | 933.79 | III-33 | 938.65 |
| I-34 | 402.50 | 4.76 | II-34 | 861.67 | III-34 | 866.53 |
| I-35 | 458.61 | 6.61 | II-35 | 973.89 | III-35 | 978.75 |
| MS-1 | 242.31 | 2.72 | | | | |
| MS-2 | 264.27 | 1.58 | | | | |
| MS-3 | 418.57 | 6.94 | | | | |
| MS-4 | 418.57 | 6.65 | | | | |

$(MS\text{-}1)_2Ni$

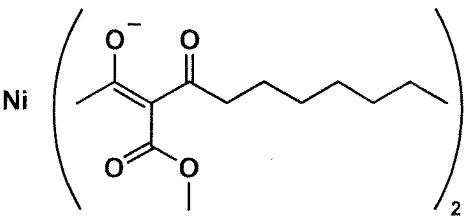

$(MS\text{-}2)_2Ni$

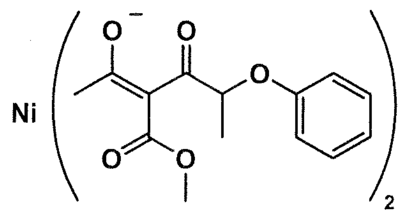

$(MS\text{-}3)_2Ni$

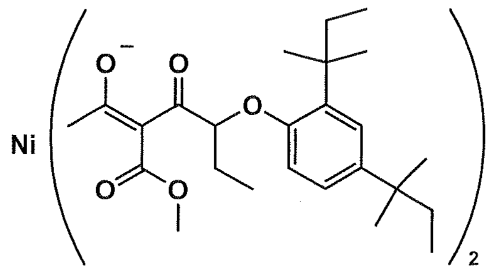

$(MS\text{-}4)_2Ni$

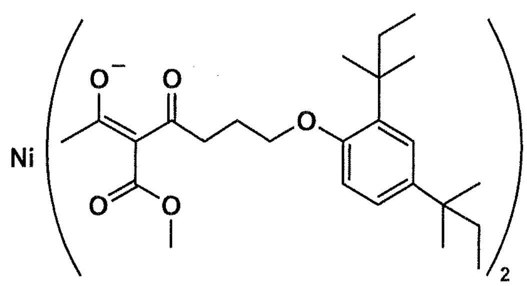

Metal containing compounds prepared by allowing the compound represented by Formula (I) to react with a divalent metal compound and metal-chelating dyes, composing the dyes shown by Formula (4), will now be described.

In Formula (1), examples of the substituents represented by $R_{11}$ and $R_{12}$ include a halogen atom, an alkyl group (having 1 - 12 carbon atoms in which the substituent linking via an oxygen atom, a nitrogen atom, a sulfur atom, or a carbonyl group is substituted, or an aryl group, an alkenyl group, an alkynyl group, a hydroxyl group, an amino group, a nitro group, a carboxyl group, a cyano group or a halogen atom may be substituted, for example, methyl, isopropyl, t-butyl, trifluoromethyl, methoxymethyl, 2-methanesulfonylethyl, 2-methanesulfonamidoethyl, or cyclohexyl); an aryl group (e.g., phenyl, 4-t-butylphenyl, 3-nitrophenyl, 3-acylaminophenyl, and 2-methoxyphenyl); a cyano group, an alkoxyl group, an aryloxy group, an acylamino group, an anilino group, a ureido group, a sulfamoylamino group, an alkylthio group, an arylthio group, an alkoxycarbonylamino group, a sulfonamido group, a carbamoyl group, a sulfamoyl group, a sulfonyl group, an alkoxycarbonyl group, a heterocyclic oxy group, an acyloxy group, a carbamoyloxy group, a silyloxy group, an aryloxycarbonylamino group, an imido group, a heterocyclic thio group, a phosphonyl group, and an acyl group.

Listed as alkyl groups and aryl groups represented by $R_{13}$ may be those represented by $R_{11}$ and $R_{12}$.

Specifically listed as 5- to 6-membered heterocycles represented by Z, which contains two carbon atoms, are rings such as benzene, pyridine, pyrimidine, triazine, pyrazine, pyridazine, pyrrole, furan, thiophene, pyrazole, imidazole, triazole, oxazole, or thiazole. These rings may further form condensed rings with other aromatic rings. There may be substituent(s) on these rings. Listed as such substituents may be the same ones as those represented by $R_1$ and $R_2$.

It is possible to produce the dyes represented by Formula (1), which are employed in the present invention,

in such a manner that, for example, the compound represented by Formula (B) below, is subjected to diazotization according to the method described in Chemical Review. Vol. 75, 241 (1975) and subsequently undergoes a prior art coupling reaction with the compounds represented by Formula (C) below.

Formula (B)

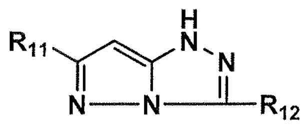

Formula (C)

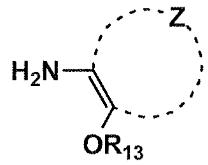

wherein each of $R_{11}$, $R_{12}$, $R_{13}$, and Z is as defined for each of $R_{11}$, $R_{12}$, $R_{13}$, and Z in Formula (1).

Representative examples of the dyes represented by Formula (1), which are employed in the present invention, will now be listed; however, the present invention is not limited thereto.

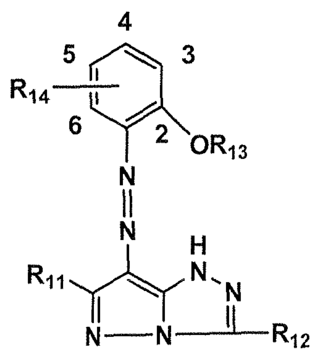

| Compound No. | $R_{11}$ | $R_{12}$ | $R_{13}$ | $R_{14}$ |
|---|---|---|---|---|
| (1)-1 | $-CH_3$ | $-C_4H_9$ | $-CH_3$ | — |
| (1)-2 | $-C_3H_7(i)$ | 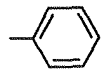 | $-CH_3$ | — |
| (1)-3 | $-C_3H_7(i)$ | $-C_2H_5$ | $-CH_3$ | — |
| (1)-4 | $-CH_3$ | $-C_2H_5$ | $-CH_3$ | — |
| (1)-5 | $-C_3H_7(i)$ | 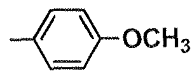 | $-CH_3$ | 4$-$Cl |
| (1)-6 | $-C_3H_7(i)$ | $-C_2H_5$ | $-CH_3$ | 4$-CO_2CH_3$ |
| (1)-7 | $-C_3H_7(i)$ | $-C_4H_9$ | $-CH_3$ | 5$-CO_2CH_3$ |
| (1)-8 | $-C_4H_9(t)$ | $-C_4H_9$ | $-CH_3$ | — |
| (1)-9 | $-C_3H_7(i)$ | 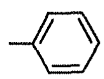 | $-C_3H_7(i)$ | — |
| (1)-10 | $-C_3H_7(i)$ | 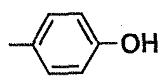 | $-CH_3$ | — |
| (1)-11 | $-C_3H_7(i)$ | $-C_3H_7$ | $-CH_3$ | 5$-$Cl |
| (1)-12 | $-C_3H_7(i)$ | 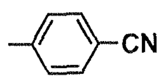 | $-CH_3$ | — |

| Compound No. | $R_{11}$ | $R_{12}$ | $R_{13}$ | $R_{14}$ |
|---|---|---|---|---|
| (1)-13 | $-C_4H_9(t)$ | 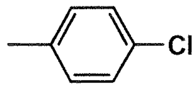 | $-CH_3$ | – |
| (1)-14 | $-SCH_3$ | 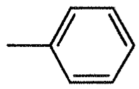 | $-CH_3$ | – |
| (1)-15 | $-NHCO$ 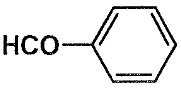 | $-C_2H_5$ | $-CH_3$ | – |
| (1)-16 | $-NH$ 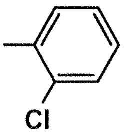 | $-C_2H_5$ | $-CH_3$ | – |
| (1)-17 | $-OCH_3$ | $-C_4H_9$ | $-CH_3$ | – |
| (1)-18 | $-C_4H_9(t)$ | $-C_4H_9$ | $-CH_3$ | $4-CO_2H$ |
| (1)-19 | $-C_3H_7(i)$ | 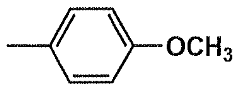 | $-CH_3$ | – |
| (1)-20 | $-C_3H_7(i)$ | 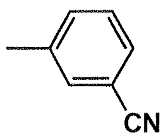 | $-CH_3$ | – |
| (1)-24 | $-C_3H_7(i)$ | $-C_2H_5$ | $-CH_3$ | $5-Cl$ |
| (1)-25 | $-C_4H_9(t)$ | $-C_4H_9$ | $-CH_3$ | $5-Cl$ |
| (1)-26 | $-C_4H_9(t)$ | $-C_2H_5$ | $-CH_3$ | $5-Cl$ |
| (1)-27 | $-C_4H_9(t)$ | $-CH_2$ 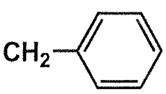 | $-CH_3$ | $5-Cl$ |
| (1)-28 | $-C_4H_9(t)$ | $-CH_2$ 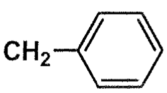 | $-CH_3$ | – |
| (1)-29 | $-C_4H_9(t)$ | $-CHC_4H_9$<br>$C_2H_5$ | $-CH_3$ | $5-Cl$ |
| (1)-30 | $-C_4H_9(t)$ | $-C_6H_{13}$ | $-CH_3$ | $5-Cl$ |
| (1)-31 | $-C_4H_9(t)$ | $-CH_3$ | $-CH_3$ | $5-Cl$ |
| (1)-32 | $-C_4H_9(t)$ | $-CH_3$ | $-CH_3$ | – |

(1)-21

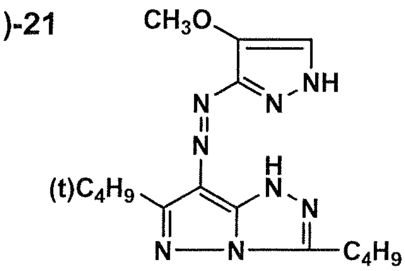

(1)-22

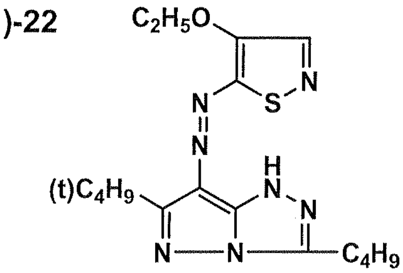

(1)-23

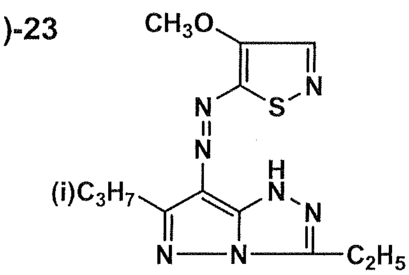

In Formula (2), $R_{21}$ represents a hydrogen atom, a halogen atom (fluorine, chlorine, etc.), or a univalent substituent. Listed as univalent substituents are an alkyl group, an aryl group, a hetero-aromatic group, an acyl group, an amino group, a nitro group, a cyano group, an acylamino group, an alkoxy group, a hydroxyl group, and an alkoxycarbonyl group, which may be further substituted.

$R_{22}$ represents an aromatic carbon ring group or a hetero-aromatic group, and preferably listed are residual groups of a benzene ring, a pyridine ring, a pyrimidine ring, a furan ring, a thiophene ring, a thiazole ring, an imidazole ring, and a naphthalene ring. These residual groups may form condensation rings with other carbon rings (for example, a benzene ring) as well as heterocycles (for example, a pyridine ring). Substituents on the ring include an alkyl group, an aryl group, an acyl group, an amino group, a nitro group, a cyano group, an acylamino group, an alkoxy group, a hydroxyl group, an alkoxycarbonyl group, and a halogen atom, which may be further substituted. X represents a methine group or a nitrogen atom.

$R_{23}$ represents Formula (3). Above Formula (3) represents a nitrogen-containing aromatic heterocyclic residual group which may have a substituent. Preferred as the above heterocycles are pyrazole, imidazole, oxazole, thiazole, isoxazole, triazole, pyridine, pyridazine, pyrimidine, pyrazine, triazine, benzimidazole, benzothiazole, benzoxazole, and isoquinoline. These rings may further form a condensed ring together with other carbon rings (for example, a benzene ring) and heterocycles (for example, a pyridine ring).

Listed as substituents on the ring are an alkyl group, an aryl group, an acyl group, an amino group, a nitro group, a cyano group, an acylamino group, an alkoxy group, a hydroxyl group, an alkoxycarbonyl group, and a halogen atom. These substituents may further be substituted. $X_1$ represents either a carbon atom or a nitrogen atom.

Y represents a group of atoms forming a nitrogen-containing aromatic heterocycle, but Y is preferably a group of atoms forming a 5- to 6-membered nitrogen containing hetero-aromatic ring, which may have a substituent on the ring.

Listed as examples of substituents on the ring may be those of preferred $R_{22}$.

It is possible to synthesize dyes represented by Formula (2), which are employed in the present invention according to conventional prior art methods. For example, it is possible to synthesize the azomethine dye in General Formal (2) according to the oxidation coupling method described in JP-A Nos. 63-113077, 3-275767, and 4-89287.

Specific examples of the dyes represented by Formula (2) will now be listed; however the present invention is not limited thereto.

**Substituent $R_{21}$**

**(1)**

$—CH_3$

**(2)**

$—C_4H_9(t)$

**(3)**

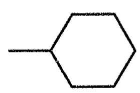

**(4)**

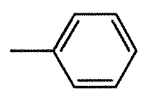

**(5)**

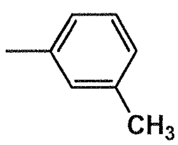

**(6)**

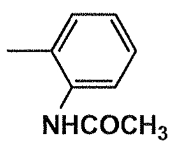

**(7)**

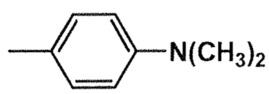

**(8)**

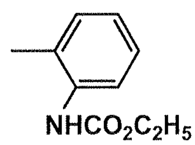

**(9)**

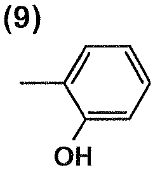

**(10)**

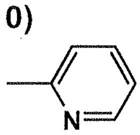

**(11)**

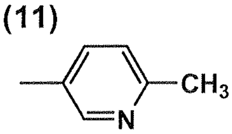

**(12)**

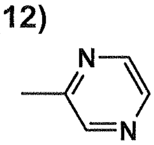

**(13)**

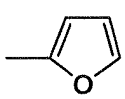

**(14)**

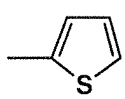

**(15)**

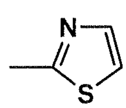

**(16)**

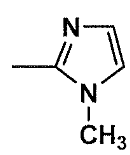

(17) 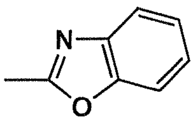

(18) 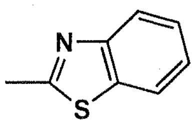

(19) 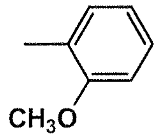

(20) 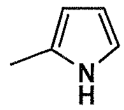

**Substituent $R_{22}$**

(1) 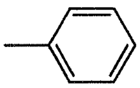

(2) 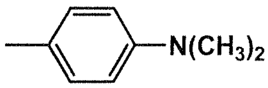

(3) 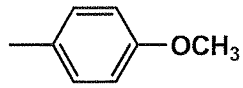

(4) 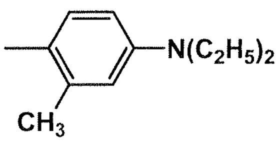

(5) 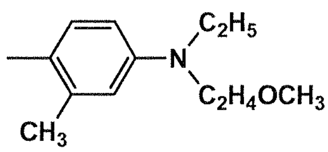

(6) 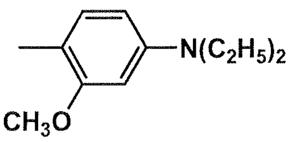

(7) 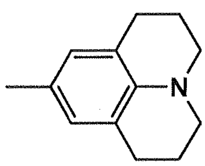

(8) 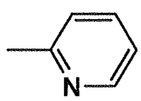

(9) 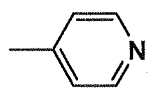

(10) 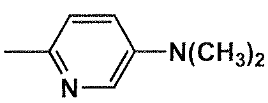

(11) 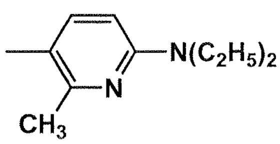

(12) 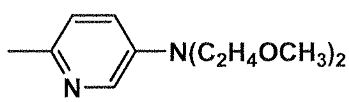

(13) 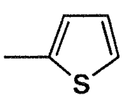

(14) 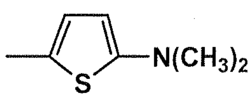

(15) 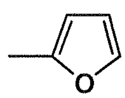

(16)

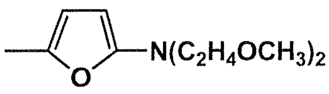

(17)

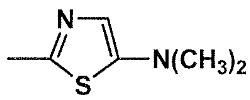

(18)

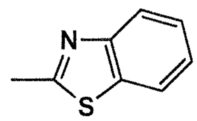

(19)

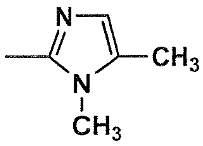

(20)

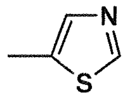

(21)

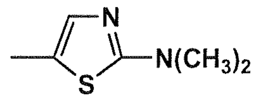

(22)

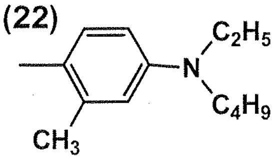

(23)

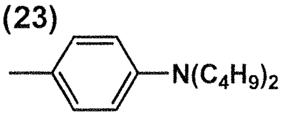

(24)

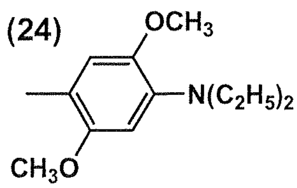

**Substituent $R_{23}$**

(1)

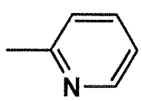

(2)

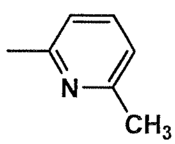

(3)

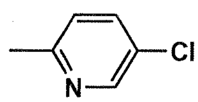

(4)

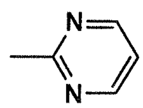

(5)

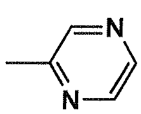

(6)

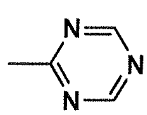

(7)

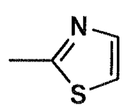

(8)

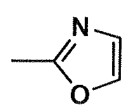

(9)

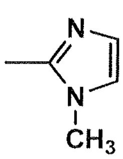

(10)

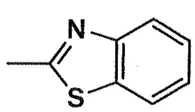

(11)

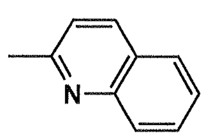

(12)

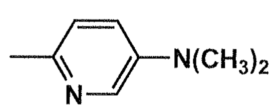

(13)

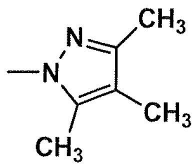

(14)

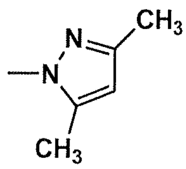

(15)

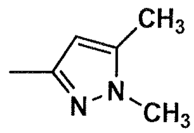

(16)

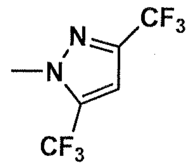

Table 2

| Dye | $R_{21}$ | $R_{22}$ | $R_{23}$ | X | Dye | $R_{21}$ | $R_{22}$ | $R_{23}$ | X |
|---|---|---|---|---|---|---|---|---|---|
| (2)-1 | (1) | (2) | (15) | N | (2)-20 | (3) | (16) | (9) | CH |
| (2)-2 | (1) | (6) | (9) | N | (2)-21 | (3) | (18) | (10) | CH |
| (2)-3 | (1) | (6) | (10) | N | (2)-22 | (4) | (3) | (2) | CH |
| (2)-4 | (1) | (11) | (7) | N | (2)-23 | (4) | (3) | (14) | N |
| (2)-5 | (1) | (11) | (8) | N | (2)-24 | (4) | (7) | (13) | N |
| (2)-6 | (1) | (17) | (8) | CH | (2)-25 | (4) | (10) | (11) | N |
| (2)-7 | (1) | (20) | (6) | CH | (2)-26 | (4) | (13) | (12) | CH |
| (2)-8 | (1) | (21) | (7) | CH | (2)-27 | (4) | (15) | (11) | CH |
| (2)-9 | (2) | (4) | (3) | N | (2)-28 | (5) | (9) | (14) | CH |
| (2)-10 | (2) | (4) | (5) | N | (2)-29 | (5) | (12) | (13) | CH |
| (2)-11 | (2) | (4) | (6) | N | (2)-30 | (5) | (21) | (12) | N |
| (2)-12 | (2) | (8) | (3) | CH | (2)-31 | (10) | (2) | (15) | N |
| (2)-13 | (2) | (10) | (4) | CH | (2)-32 | (16) | (13) | (15) | CH |
| (2)-14 | (2) | (11) | (1) | N | (2)-33 | (17) | (18) | (15) | N |
| (2)-15 | (2) | (13) | (15) | CH | (2)-34 | (18) | (21) | (15) | CH |
| (2)-16 | (2) | (14) | (1) | CH | (2)-35 | H | (7) | (16) | CH |
| (2)-17 | (2) | (14) | (4) | N | (2)-36 | H | (16) | (16) | N |
| (2)-18 | (2) | (19) | (5) | CH | (2)-37 | (2) | (4) | (5) | CH |
| (2)-19 | (3) | (5) | (2) | N | (2)-38 | (2) | (22) | (5) | CH |

In Formula (4), $R_{31}$ and $R_{32}$ each represent a substituted or unsubstituted aliphatic group, which may be the same or different. Listed as examples of aliphatic groups may be an alkyl group, a cycloalkyl group, an alkenyl group, and an alkynyl group. Examples of the alkyl groups include a methyl group, an ethyl group, a propyl group, and an i-propyl group. Listed as groups capable of substituting these alkyl groups are a straight or branched alkyl group (e.g., a methyl group, an ethyl group, an i-propyl group, a tertiary butyl group, an n-dodecyl group, and a 1-hexyknonyl group); a cycloalkyl group (e.g., a cyclopropyl group, a cyclohexyl group, a bicyclo[2.2.1]heptyl group, and an adamantyl group); an alkenyl group (e.g., a 2-propylene group) and an oleyl group); an aryl group (e.g., a phenyl group, an ortho- tolyl group, an ortho-anisyl group, a 1-naphthyl group, and a 9-anthranyl group); a heterocyclic group (e.g., a 2-tetrahydrofuryl group, a thiophenyl group, a 4-imidazolyl group, and a 2-pyridyl group); a halogen atom (e.g., a fluorine atom, a chlorine atom, and a bromine atom); a cyano group, a nitro group, a hydroxyl group; a carbonyl group (e.g., an alkylcarbonyl group such as an acetyl group, a trifluoroacetyl group, or a pivaloyl group, and an arylcarbonyl group such as a benzoyl group, a pentafluorobenzoyl group, or a 3,5-di-t-butyl-4-hydroxybenzoyl group); an oxycarbonyl group (e.g., an alkoxycarbonyl group such as a methoxycarbonyl group, a cyclohexyloxycarbonyl group, or an n-dodecyloxycarbonyl group, and an aryloxycarbonyl group such as a phenoxycarbonyl group, a 2,4-di-t-amylphenoxycarbonyl group, or a 1-naphthyloxycarbonyl group, a heterocyclic oxycarbonyl group such as a 2-pyridyloxycarbonyl group, a 1-phenylpyrazolyl-5-oxycarbonyl group); a carbamoyl group (e.g., an alkylcarbamoyl group such as a dimethylcarbamoyl group, a 4-(2,4-di-t-smylphenoxy)butylaminocarbonyl group, and an arylcarbamoyl group such as a phenylcarbamoyl group, a 1-naphthylcarbamoyl group); an alkoxy group (e.g., a methoxy group and a 2-ethoxy group), an aryloxy group (e.g., a phenoxy group, a 2,4-di-amylphenoxy group, a 4-(hydroxyphenylsulfonyl)phenoxy group); a heterocyclic oxy group (e.g., a 4-pyridyloxy group and a 2-hexahydropyranyloxy group); a carbonyloxy group (e.g., an

alkylcarbonyloxy group such as an acetyloxy group, a trifluoroacetyloxy group, or a pivaloyloxy group, and an aryloxy group such as a benzoyloxy group or a pentafluorobenzoyloxy group); a urethane group (e.g., an alkylurethane group such as an N-dimethylurethane) and an arylurethane group such as an N-phenylurethane group and an N-(p-cyanophenyl)urethane group); a sulfonyloxy group (e.g., an alkylsulfonyloxy group such as a methanesulfonyloxy group, a trifluoromethanesulfonyloxy group, or an n-dodecanesulfonyloxy group) and an arylsulfonyloxy group such as a benznesulfonyloxy group, a p-toluenesulfonyloxy group); an amino group (e.g., an alkylamino group such as a dimethylamino group, a cyclohexylamino group, or an n-dodecylamino group) and an arylamino group such as an anilino group or a p-t-octylanilino group); a sulfonylamino group (e.g., an alkylsulfonylamino group such as a methanesulfonylamino group, a heptafluoropropanesulfonylamino group, or an n-hexadecylsulfonylamino group and an arylsulfonylamino group such as a p-toluenesulfonylamino group or a pentafluorobenzenesulfonylamino group); a sulfamoylamino group (e.g., an alkylsulfamoylamino group such as an N,N-dimethylsulfamoylamino group and an arylsulfamoylamino group such as an N-phenylsulfamoylamino group); an acylamino group (e.g., an alkylcarbonylamino group such as an acetylamino group or a myristoylamino group and a benzoylamino group such as an arylcarbonylamino group); a ureido group (e.g., an alkylureido group such as an N,N-dimethylaminoureido group and a arylureido group such as an N-phenylureido group, an N-(p-cyanophenyl)ureido group); a sulfonyl group (e.g., an alkylsulfonyl group such as a methanesulfonyl group or a trifluoromethanesulfonyl group, and an arylsulfonyl group such as a p-toluenesulfonyl group); a sulfamoyl group (e.g., an alkylsulfamoyl group such as a dimethylsulfamoyl group or a 4-(2,4-di-amylphenoxy) butylaminosulfamoyl group and an arylsulfamoyl group such as a phenylsulfamoyl group); an arylthio group (e.g., a phenylthio group); and a heterocyclic thio group (e.g., a 1-phenyltretrazole-5-thio group and a 5-methyl-1,3,4-oxadiazole-2-thio group.

Listed as examples of cycloalkyl groups as well as alkenyl groups are those identical to the above substituents. Further, examples of alkynyl groups include 1-propyne, 2-butyne, and 1-hexyne.

It is also preferable employing $R_{31}$ and $R_{32}$ to form a non-aromatic ring structure (for example, a pyrrolidine ring, a piperidine ring, and a morpholine ring).

Of the above substituents, $R_{33}$ preferably represents an alkyl group, a cycloalkyl group, an alkoxy group, and an acylamino group. Further, n represents an integer of 0 - 4 and when n is at least 2, a plurality of $R_{33}$ may be the same or different.

$R_{34}$ represents an alkyl group, examples of which include a methyl group, an ethyl group, an i-propyl group, a t-butyl group, an n-dodecyl group, and a 1-hexylnonyl group. $R_{34}$ is preferably a secondary or tertiary alkyl group, and examples of preferred secondary or tertiary alkyl groups include an isopropyl group, a sec-butyl group, a tert-butyl group, and a 3-heptyl group. The most preferred substituents as $R_{34}$ include an isopropyl group and a tert-butyl group. The alkyl groups represented by $R_{34}$ may be substituted, but are only substituted with a substituent comprising carbon atoms and hydrogen atoms. They are not substituted with substituents having other atoms.

$R_{35}$ represents an alkyl group, of which examples include an n-propyl group, an i-propyl group, a t-butyl group, an n-dodecyl group, and a 1-hexylnonyl group. $R_{35}$ is preferably a secondary or tertiary alkyl group. Listed as examples of preferred secondary or tertiary alkyl groups are an isopropyl group, a sec-butyl group, a tert-butyl group, and a 3-heptyl group. The most preferred substituents as $R_{34}$ include an isopropyl group and a ter-t-butyl group. The alkyl groups represented by $R_{34}$ may be substituted, but are substituted with a substituent composed only of carbon atoms and hydrogen atoms. They are not substituted with substituents having other atoms.

$R_{36}$ represents an alkyl group, and the examples include an n-propyl group, an n-butyl group, an n-pentyl group, an n-hexyl group, an n-heptyl group, an iso-propyl group, a sec-butyl group, a tert-butyl group, and a 3-heptyl group. Particularly preferred substituents as $R_{36}$ are straight chain alkyl groups having at least three carbon atoms. Examples of these include an n-propyl group, an n-butyl group, an n-pentyl group, an n-hexyl group, and an n-heptyl group, of which an n-propyl group and an n-butyl group are most preferred. Incidentally, the alkyl group represented by $R_{36}$ may be substituted. However, it is substituted by substituents composed only of carbon atoms and hydrogen atoms and is not substituted by substituents containing other atoms.

It is possible to synthesize the dyes represented by Formula (4) employed in the present invention, according to conventional prior art methods. It is possible to synthesize them according to the oxidation coupling method described, for example, in JP-A Nos. 2001-334755 and 2002-234266.

Specific examples of the dyes represented by Formula (4) will now be listed below; however, the present invention is not limited thereto.

(4)-1

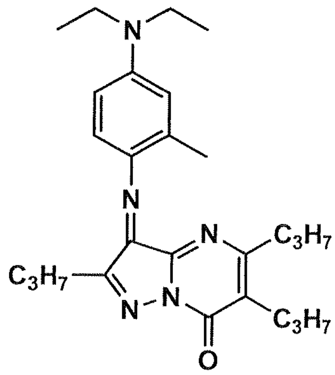

(4)-2

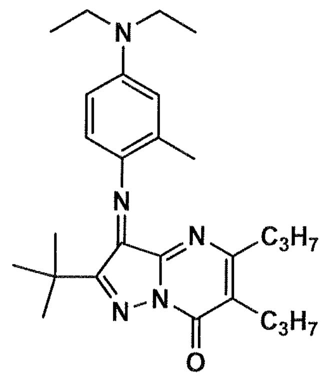

(4)-3

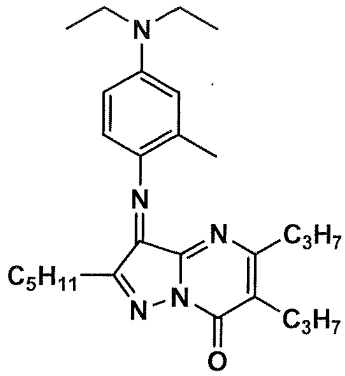

(4)-4

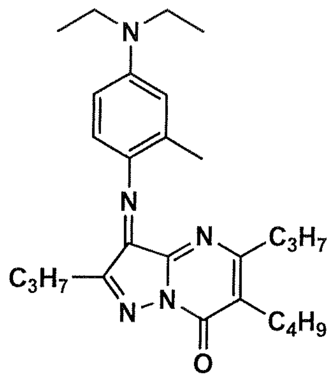

(4)-5

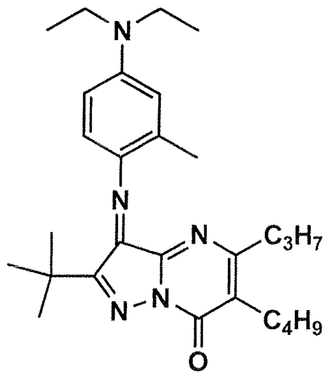

(4)-6

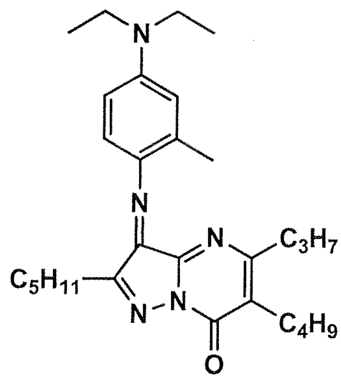

**(4)-7**

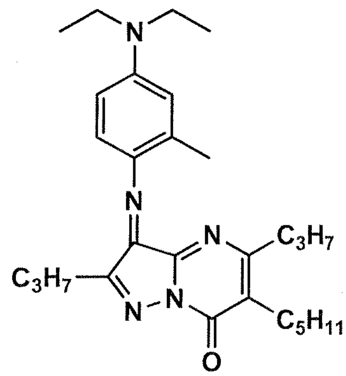

**(4)-8**

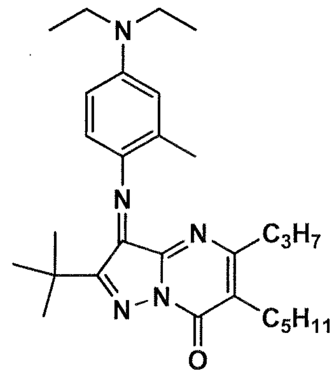

**(4)-9**

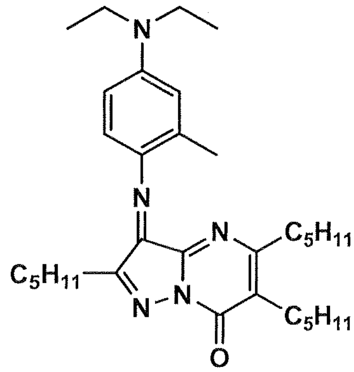

**(4)-10**

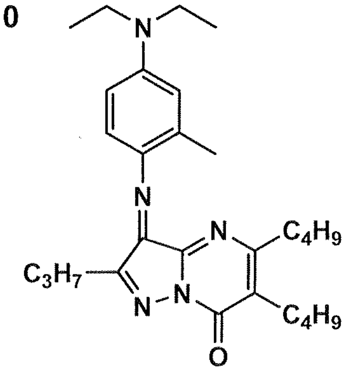

**(4)-11**

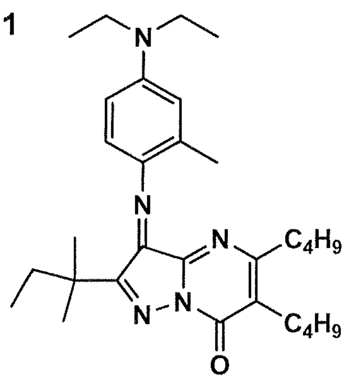

**(4)-12**

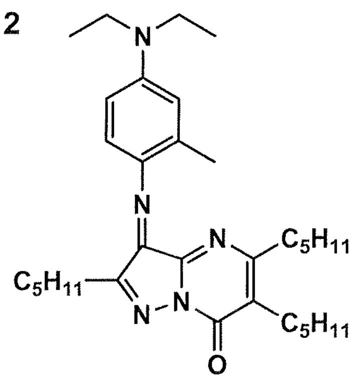

(4)-13

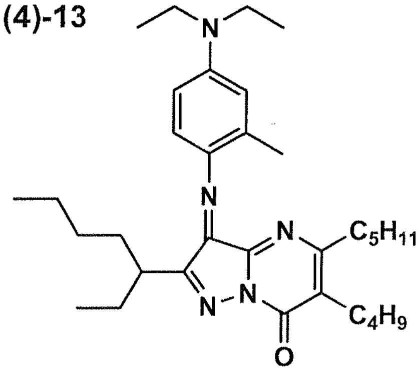

(4)-14

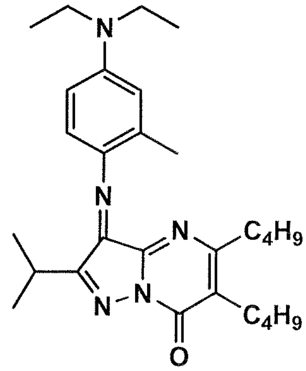

(4)-15

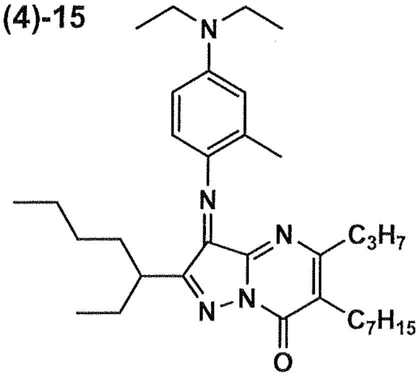

(4)-16

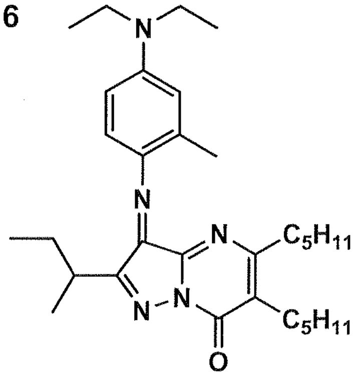

(4)-17

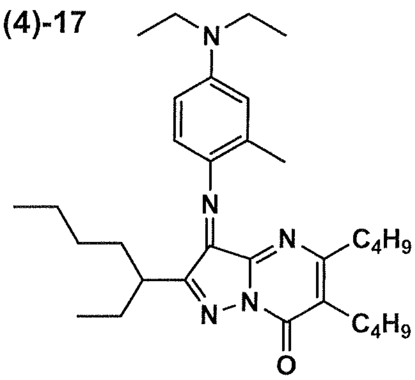

(4)-18

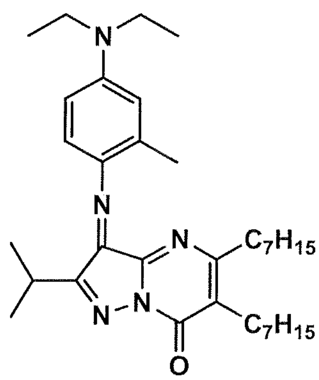

(4)-19

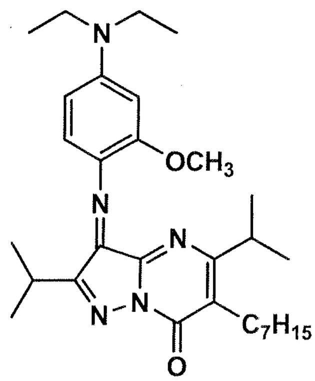

(4)-20

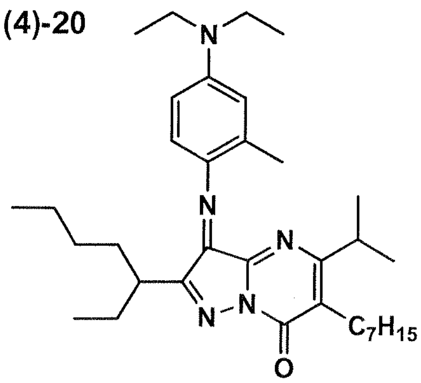

(4)-21

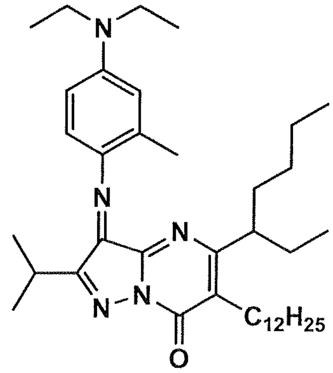

(4)-22

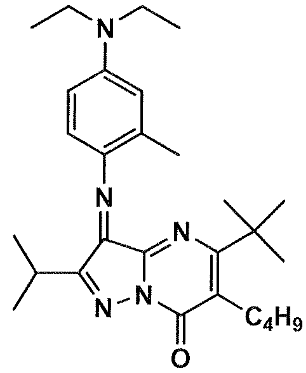

(4)-23

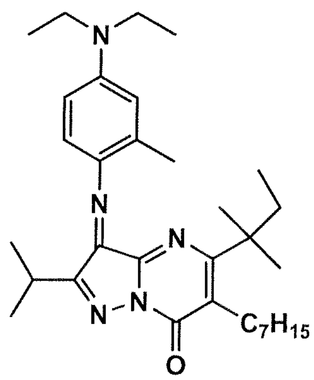

(4)-24

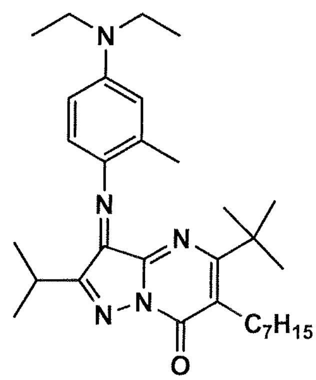

(4)-25

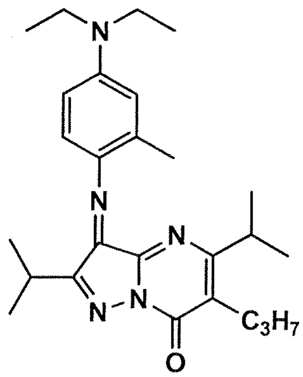

(4)-26

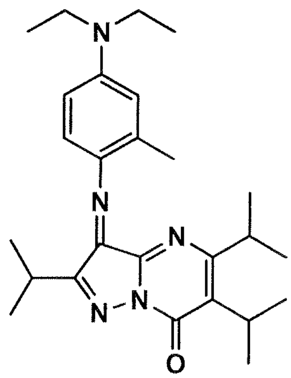

(4)-27

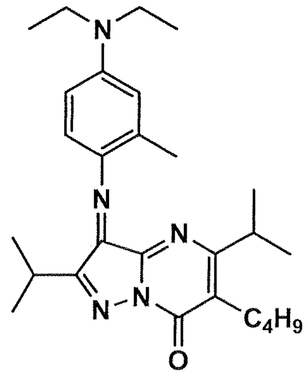

(4)-28

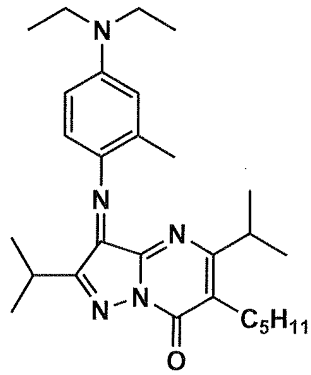

(4)-29

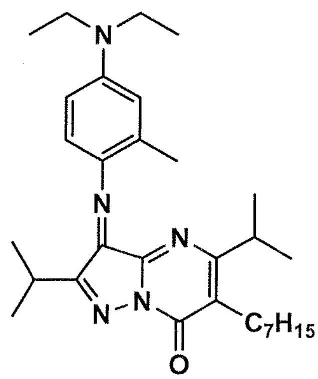

(4)-30

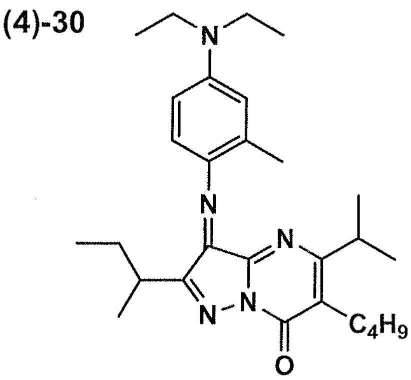

(4)-31

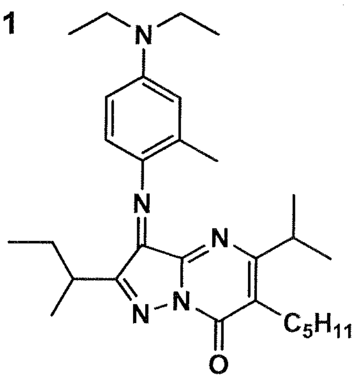

(4)-32

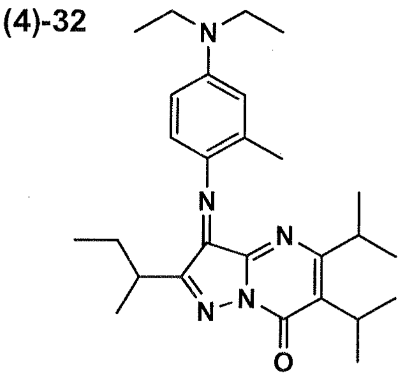

(4)-33

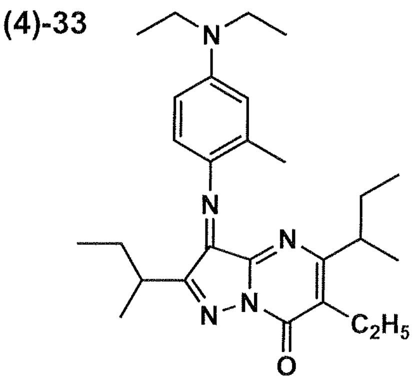

(4)-34

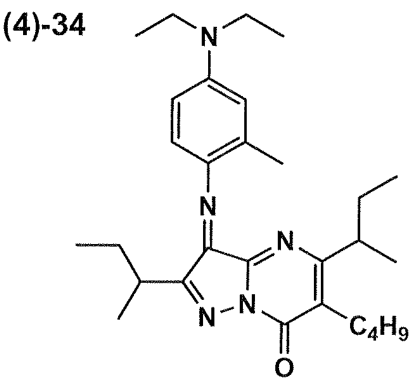

(4)-35

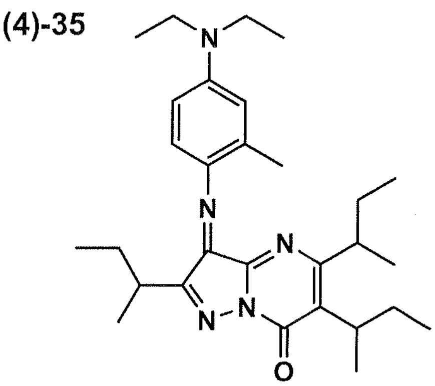

(4)-36

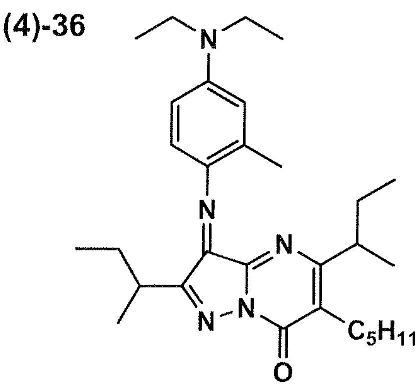

Metal-chelated dyes derived from a metal containing compound prepared by allowing the compound represented by Formula (I) to react with a divalent metal compound, and the dyes represented by Formula (1), (2), or (4) are represented by Formulas (5), (6) and (7).

Formula (5)

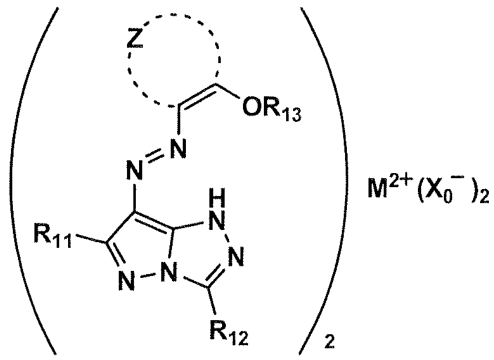

Formula (6)

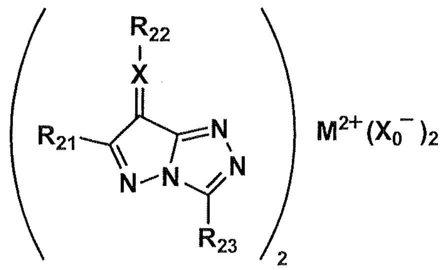

Formula (7)

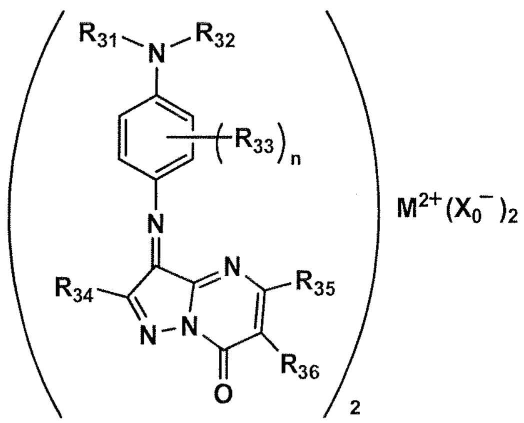

In Formulas (5) through (7), $R_{11}$, $R_{12}$, $R_{13}$, $R_{21}$, $R_{22}$, $R_{23}$, $R_{31}$, $R_{32}$, $R_{33}$, $R_{34}$, $R_{35}$, and $R_{36}$, as well as X, Z, and n are defined as in above Formulas (1), (2), and (4).

Further, $M^{2+}$ represents a divalent metal ion. Of those metal ions, in view of the color of metal incorporating compounds themselves and of chelated dyes, nickel and zinc are preferred. $X_0$ represents the compounds represented by aforesaid Formula (I), capable of forming a complex with divalent metal ions.

Specific examples of metal-chelated dyes represented by Formulas (5) through (7) are listed below; however, the present invention is not limited thereto.

Table 3

| Chelating Dye | Dye | $M^{2+}$ | Compound Represented by Formula (I) |
|---|---|---|---|
| (5)-1 | (1)-1 | $Ni^{2+}$ | I-8 |
| (5)-2 | (1)-1 | $Ni^{2+}$ | I-19 |
| (5)-3 | (1)-1 | $Ni^{2+}$ | I-22 |
| (5)-4 | (1)-1 | $Cu^{2+}$ | I-8 |
| (5) -5 | (1)-31 | $Ni^{2+}$ | I-8 |
| (5)-6 | (1)-31 | $Ni^{2+}$ | I-19 |
| (5)-7 | (1)-31 | $Ni^{2+}$ | I-22 |
| (5)-8 | (1) -31 | $Cu^{2+}$ | I-8 |
| (5)-9 | (1)-32 | $Ni^{2+}$ | I-8 |
| (5)-10 | (1)-32 | $Ni^{2+}$ | I-19 |
| (5)-11 | (1)-32 | $Ni^{2+}$ | I-22 |
| (5)-12 | (1)-32 | $Cu^{2+}$ | I-8 |
| (6) -1 | (2) -10 | $Ni^{2+}$ | I-8 |
| (6)-2 | (2)-10 | $Ni^{2+}$ | I-19 |
| (6)-3 | (2)-10 | $Ni^{2+}$ | I-22 |
| (6)-4 | (2)-10 | $Cu^{2+}$ | I-8 |
| (6)-5 | (2)-13 | $Ni^{2+}$ | I-8 |
| (6)-6 | (2)-13 | $Ni^{2+}$ | I-19 |
| (6)-7 | (2)-13 | $Ni^{2+}$ | I-22 |
| (6)-8 | (2)-13 | $Cu^{2+}$ | I-8 |
| (6)-9 | (2)-38 | $Ni^{2+}$ | I-8 |
| (6)-10 | (2)-38 | $Ni^{2+}$ | I-19 |
| (6)-11 | (2) -38 | $Ni^{2+}$ | I-22 |
| (6)-12 | (2)-38 | $Cu^{2+}$ | I-8 |
| (7)-1 | (4)-14 | $Ni^{2+}$ | I-8 |
| (7)-2 | (4)-14 | $Ni^{2+}$ | I-19 |
| (7)-3 | (4)-14 | $Ni^{2+}$ | I-22 |
| (7)-4 | (4)-14 | $Cu^{2+}$ | I-8 |
| (7)-5 | (4)-22 | $Ni^{2+}$ | I-8 |
| (7)-6 | (4)-22 | $Ni^{2+}$ | I-19 |
| (7)-7 | (4)-22 | $Ni^{2+}$ | I-22 |
| (7)-8 | (4)-22 | $Cu^{2+}$ | I-8 |
| (7)-9 | (4)-27 | $Ni^{2+}$ | I-8 |
| (7)-10 | (4)-27 | $Ni^{2+}$ | I-19 |
| (7)-11 | (4)-27 | $Ni^{2+}$ | I-22 |
| (7)-12 | (4)-27 | $Cu^{2+}$ | I-8 |

It is possible to use the chelating dyes employed in the present invention in various aspects such as thermal transfer, ink jet ink, or toner. Employed as dyes for thermal transfer may be those according to the methods described in JP-A Nos. 9-143382 and 10-265690. Further, employed as dyes for ink jet ink may be those according to the methods described in JP-A Nos. 2000-160130, 2000-315813, and 2000-315814. Still further, employed as dyes for toner may be those according to the methods described in JP-A Nos. 10-265690 and 2000-345059. However, the uses and methods of the dyes of the present invention are not limited thereto.

Synthesis Example-2

Synthesis examples of the chelated dyes of the present invention will now be described.

Synthesis Example 7 (Synthesis of Chelated Dye (5)-13)

Added to 100 ml of methanol were 20 g (0.06402 mol) of Compound (1)-32 and 28.6 g (0.03201 mol) of Metal Complex IV-9, and subsequently, while stirring, the resulting mixture was refluxed over a period of two hours. After cooling the reaction liquid composition, the solvent was distilled out by vacuum concentration. Diisopropyl ether was added to the resulting reaction mixture and deposited crystals were collected by filtration, whereby 37.0 g (at a yield of 76.2 percent) of Chelated Dye (5)-13 was obtained. The λmax of the resulting dye in acetone was 456 nm.

Synthesis Example 8 (Synthesis of Chelated Dye (5)-15)

Chelated Dye (5)-15 of 35.3 g (at a yield of 72.4 percent) was obtained performing the reaction and purification employing the same formulation as for Synthesis Example 7, except that Metal Complex IV-9 was replaced with 28.8 g (0.03201 mol) of V-9. The λmax of the resulting dye in acetone was 445 nm.

Synthesis Example 9 (Synthesis of Chelated Dye (6)-13)

Added to 100 ml of methanol were 20 g (0.04499 mol) of Compound (2)-38 and 28.6 g (0.03201 mol) of Metal Complex IV-9, and subsequently, while stirring, the resulting mixture was refluxed over a period of two hours. After cooling the reaction liquid composition, deposited solids were collected by filtration, whereby 43 g of crude crystals was obtained. The resulting crude crystals were recrystallized employing methanol, whereby 35.3 g (at a yield of 76.2 percent) of Chelated Dye (6)-13 was obtained. The λmax of the resulting dye in acetone was 546 nm.

Synthesis Example 10 (Synthesis of Chelated Dye (6)-16)

Chelated Dye (6)-16 of 32.4 g (at a yield of 81.9 percent) was obtained performing the reaction and purification employing the same formulation as for Synthesis Example 9, except that Metal Complex IV-9 was replaced with 19.5 g (0.032249 mol) of VI-8. The λmax of the resulting dye in acetone was 547 nm.

Synthesis Example 11 (Synthesis of Chelated Dye (7)-13)

Added to 100 ml of methanol were 20 g (0.04448 mol) of Compound (4)-27 and 19.9 g (0.02224 mol) of Metal Complex IV-9, and subsequently, while stirring, the resulting mixture was refluxed over a period of two hours. After cooling the reaction liquid composition, the solvent was distilled out by vacuum concentration. Ethyl acetate was added to the resulting reacted mixture, and deposited crystals were collected by filtration, whereby 31.5 g (at a yield of 79.1 percent) of Chelated Dye (7)-13 was obtained. The λmax of the resulting dye in acetone was 626 nm.

Synthesis Example 12 (Synthesis of Chelated Dye (7)-16)

Chelated Dye (7)-16 of 34.0 g (at a yield of 86.6 percent) was obtained performing the reaction and purification employing the same formulation as for Synthesis Example 11, except that Metal Complex IV-9 was replaced with 19.3 g (0.02249 mol) of VI-8. The λmax of the resulting dye in acetone was 627 nm.

The thermal transfer recording material of the present invention includes an ink sheet and a thermal transfer image receptive sheet. The ink sheet, thermal transfer image receptive sheet, and thermal transfer recording method of the present invention will now be described.

(Ink Sheet: Support)

Without modification, employed as supports may be the same as those which are employed as conventional thermal transfer sheets, and the above supports are not particularly limited. Specific examples of preferred supports include thin paper such as glassine paper, condenser paper, or paraffin paper; high heat resistant polyester such as polyethylene terephthalate, polyphenylene sulfide, polyether ketone, or polyether sulfone; and oriented or non-oriented film of plastics such as polypropylene, polycarbonate, cellulose acetate, polyethylene derivatives, polyvinyl chloride, polyvinylidene chloride, polystyrene, polyamide, polyimide, polymethylpentane, or ionomer, as well as lamination products thereof.

It is possible to suitably select the thickness of the above supports depending on materials so that the resulting strength, heat conductivity, and heat resistance become optimal. However, those at a thickness between about 1 - about 100 µm are preferably employed. In the case in which supports, as described above, exhibit insufficient adhesion to the dye layer formed thereon, it is preferable that the surface is subjected to a primer treatment or corona treatment.

(Ink Sheet: Dye Layer)

In the present invention, from the viewpoint of achieving the desired storage stability of images, it is preferable that as heat diffusible dyes incorporated in the dye layer provided on one side of a support, either chelating dyes or post-chelate dyes (or called as chelate forming dyes) are combined with metal sources.
Listed as post-chelate dyes may be the compounds represented by Formulas (1), (2), and (4). However, they are not particularly limited as long as they are thermally transferable, and it is possible to select various prior art compounds and simultaneously used. It is possible to use the cyan dyes, magenta dyes, and yellow dyes described, for example, in JP-A Nos. 10-258580, 2000-1057, and 2002-234266.
The added amount of dyes employed in the present invention is preferably 0.1 - 20 g/$m^2$, but is more preferably 0.2 - 5 g/$m^2$.
Binder resins employed in the dye layer include watersoluble polymers such as cellulose based, polyacrylic acid based, polyvinyl alcohol based, or polyvinylpyrrolidone based resins, as well as organic solvent-soluble organic polymers such as acryl resins, methacryloyl resins, polystyrene, polycarbonate, polysulfone, polyethersulfone, polyvinyl butyral, polyvinyl acetal, or ethylcellulose. Of these resins, preferred are polyvinyl butyral, polyvinyl acetal, or cellulose based resins, which result in the desired storage stability. In cases in which organic solvent-soluble polymers are employed, one or more than two types may be employed to be dissolved in organic solvents, and in addition, may be employed in the form of a latex dispersion. The amount of binder resins used is preferably 0.1 - 50 g per $m^2$ of the support.
Further, in order to enhance releasing properties, releasing agents may be added, or a leasing layer may be provided. Employed as releasing agents may be reactive curing type silicone, phosphoric acid based surface active agents, and fluorine compounds. The amount of releasing agents used is preferably 0.5 - 40 percent by weight with respect to solids in the layer in which they are incorporated. Further, in cases in which a releasing layer is provided, it is possible to use binders which are the same as those employed in the above dye layer.

(Ink Sheet: BC Layer)

In order to provide heat resistance, it is preferable to provide a backing layer on the surface opposite the surface of a support on which the dye layer is provided.

(Ink Sheet: Protective Layer)

In thermal transfer recording of the present invention, in recording media, it is possible to provide a transparent layer which is formed by thermal transfer on the surface after dyes are transferred.
The protective layer employed in the present invention may be provided on the same surface as for the above dye layer, namely the layer order. When only the protective layer is employed as a protective transfer sheet, it is possible to use the same as those above as a support and a backing layer.
In the present invention, it is preferable to provide a thermal transfer protective layer on a support via a non-transferable releasing layer.
In order that the adhesion force between the support and the non-transferable releasing layer is sufficiently higher than that between the non-transferable releasing layer and the thermal transfer protective layer, and the adhesion force between non-transferable releasing layer and the thermal transfer protective layer prior to application of heat is higher than that after application of heat, it is preferable that:

(1) minute inorganic particles at a particle diameter of at most 40 nm are incorporated together with resinous binders,
(2) alkyl vinyl ether-maleic anhydride copolymers and derivatives thereof or a mixture thereof are incorporated in a total amount of at least 20 percent by weight, or
(3) ionomers are incorporated in an amount of at least 20 percent by weight. If desired, other additives may be incorporated in the non-transferable releasing layer.

Employed as minute inorganic particles may be, for example, minute silica particles such as dehydrated silica or colloidal silica, and particles of metal oxides such as tin oxide, zinc oxide, or zinc antimonate. The diameter of minute inorganic particles is preferably at most 40 nm. When the diameter exceeds 40 nm, the unevenness of the surface of the thermal transfer protective layer increases due to the unevenness of the surface of the releasing layer, whereby the transparency of the protective layer is undesirably degraded.
Resinous binders which are mixed with minute inorganic particles are not particularly limited, and it is possible to use any of the mixable resins. Examples include polyvinyl alcohol resins (PVA) at various degrees of saponification,

polyvinyl acetal resins, polyvinyl butyral resins, acryl based resins, polyamide based resins, cellulose based resins such as cellulose acetate, alkyl cellulose, carboxymethyl cellulose, or hydroxyalkyl cellulose, and polyvinylpyrrolidone resins.

The blending ratio (minute inorganic particles/other blending components) of minute inorganic parties to other blending components comprising resinous binders as a major component is controlled preferably in the range of 30 : 70 - 80 : 20 in terms of weight. When the blending ratio is less than 30 : 70, effects of the minute inorganic particles are not sufficiently exhibited, while when the ratio exceeds 80 : 20, the releasing layer does not complete a layer, whereby portions are generated in which the support is brought into direct contact with the protective layer.

Employed as alkyl vinyl ether-maleic anhydride copolymers or derivatives thereof may, for example, be those in which the alkyl group of the alkyl vinyl ether portion is either a methyl group or an ethyl group, or in which the portion of malic anhydride partially or completely forms a half-ester with alcohol (for example, methanol, ethanol, propanol, isopropanol, butanol, or isobutanol).

The releasing layer may be formed employing only alkyl vinyl ether-maleic anhydride copolymers and derivatives thereof, or mixtures thereof. In order to control the delamination strength, other resins or minute particles may further be added. In such cases, it is preferable that at least 20 percent by weight of alkyl vinyl ether-maleic anhydride copolymers and derivative thereof, or mixtures thereof, are incorporated in the releasing layer. When the content is less than 20 percent, it becomes impossible to sufficiently exhibit the effects of alkyl vinyl ether-maleic anhydride copolymers and derivatives thereof.

Either resins or minute particles blended with alky vinyl ether-maleic anhydride copolymers or derivatives thereof are not particularly limited, as long as they are mixable and result in desired layer transparency during layer formation and it is possible to use any of the appropriate materials. For example, preferably employed are the aforementioned minute inorganic particles and resinous binders which are mixable with minute inorganic particles.

Employed as ionomers may, for example, be Surlyn A (produced by DuPont), as well as the Chemipearl S Series (produced by Mitsui Petrochemical Industries, Ltd.). Further, it is possible to further add to ionomers, for example, the aforementioned minute inorganic partials, resinous binders mixable with minute inorganic particles, or other resins and minute particles.

The non-transferable releasing layer is formed in such a manner that a liquid coating composition comprising any of above components (1) through (3) at the predetermined ratio is prepared, the resulting liquid coating composition is applied onto a support employing prior art techniques such as a gravure coating method or a gravure reverse coating method, and subsequently, the coating is dried. The thickness of the non-transferable releasing layer is commonly controlled to be about 0.1 - about 2 μm after drying.

The thermal transfer protective layer applied onto the support via or not via a non-transferable releasing layer may be in the form of a multilayered structure. In the case in which a multilayer is structured, other than the protective layer which is mainly employed to provide various types of durability with images, an adhesion layer which is arranged on the uppermost surface of the thermal transfer protective layer to enhance adhesion between the thermal transfer protective layer and the image receptive surface of printing materials, an auxiliary protective layer, and a layer (for example, an anti-counterfeiting layer and a hologram layer) which adds functions other than genuine functions of the protective layer may be provided. The order of the main protective layer and other layer(s) is optional. However, commonly, other layer(s) are arranged between the adhesion layer and the main protective layer so that the main protective layer becomes the uppermost surface of the image receptive surface after image transfer.

It is possible to form the main protective layer which is one of the multilayered thermal transfer protective layers or the thermal transfer protective layer in a single layer structure, employing various types of resins which are conventionally known as protective layer forming resins. As protective layer forming resins, it is possible to exemplify polyester resins, polystyrene resins, acryl resins, polyurethane resins, acryl-urethane resins, resins which are prepared by modifying these resins with silicone, and mixtures thereof, as well as ionizing radiation curable resins and ultraviolet radiation shielding resins.

A protective layer containing ionizing radiation curable resins exhibits excellent plasticizer resistance as well as abrasion resistance. Employed as ionizing radiation curable resins are those known in the art. For example, it is possible to use those which are prepared in such a manner that radically polymerizable polymers or oligomers are subjected to crosslinking and curing while exposed to ionizing radiation, if desired, photopolymerization initiators are added, and the resulting mixture undergoes polymerization crosslinking employing electron beams or ultraviolet radiation.

The major purpose of the protective layer containing ultraviolet radiation shielding agents is to provide printed matter with light-fastness. Employed as ultraviolet radiation shielding resins are, for example, resins prepared by allowing reactive ultraviolet absorbing agents to react with thermoplastic resins or the above ionizing radiation curable resins. More specifically, it is possible to exemplify those prepared in such a manner that reactive groups such as addition polymerizable double bond having groups (for example, a vinyl group, an acryloyl group, and an methacryloyl group), an alcoholic hydroxyl group, an amino group, a carboxyl group, an epoxy group or an isocyanate group are

introduced into conventional prior art non-reactive organic ultraviolet radiation absorbing agents such as salicylate based, benzophenone based, benzotriazole based, substituted acrylonitrile based, nickel chelate based, or hindered amine based ones.

The main protective layer arranged in a single layer-structured or a multilayer-structured thermal transfer protective layer commonly results in a thickness of about 0.5 - abut 10 μm, though it may vary depending on the types of protective layer forming resins.

An adhesion layer may be formed on the uppermost surface of the thermal transfer protective layer. It is possible to form the adhesion layer employing resins such as acryl resins, vinyl chloride based resins, vinyl acetate based resins, vinyl chloride/vinyl acetate copolymer resins, polyester based resins, or polyamide based resins, which exhibit excellent adhesion during heating. In addition to the above resins, if desired, mixed may be the aforementioned ionizing radiation curable resins and ultraviolet radiation shielding resins. The thickness of the adhesion layer is commonly set at 0.1 - 5 μm.

In order to form such a thermal transfer protective layer on a non-transferable releasing layer or a support, for example, a protective layer forming liquid coating composition containing protective layer forming resins, an adhesion layer forming liquid coating composition containing heat adhesive resins, and liquid coating compositions to form layers, which are added if needed, are previously prepared, and those liquid coating compositions are applied onto a non-transferable releasing layer or a support in the specified order, and subsequently dried. Each of the liquid coating compositions may be applied according to conventional methods known in the art. An appropriate primer layer may be provided between each of the layers.

The above-described thermal transfer protective layer may contain a metal-containing compound according to the requirement. The amount of the metal-containing compound to be incorporated is not specifically limited, however, the preferred amount is 0.01 to 40 weight% based on the total weight of the protective layer.

(Thermal Transfer Image Receptive Sheet: Metal Source Layer)

In the thermal transfer image receptive sheet of the present invention, a liquid coating composition, which is prepared by employing resins which are similar to binder resins employed in the dye layer in which metal containing compounds are employed which are prepared by allowing the compound represented by Formula (I) of the present invention to react with a divalent metal salt, is applied onto the support described below and subsequently dried. The resulting coating is designated as a metal source layer.

The added amount of metal sources is preferably 0.01 - 1 percent by weight with respect to the total solids of the resinous layer (when provided as a dye fixer containing layer, its solid is included), but is more preferably 0.05 - 0.5 percent by weight. When the amount is at most 0.01 percent by weight, the resulting addition effects is small, while when it is at least 1 percent by weight, generation of kick-back is pronounced.

(Thermal Transfer Image Receptive Layer: Interlayer)

At least one interlayer may be arranged between the image receptive layer and the support. "Interlayer" refers to all layers arranged between the image receptive layers, such as an adhesion layer (being a primer layer), a barrier layer, an ultraviolet radiation absorbing layer, a foaming layer, or an antistatic layer, and the support, and if desired, any of the prior art layers may be employed. Further, in order to conceal glittering and non-uniformity of supports, the addition of white pigments, such as titanium oxide, to the interlayer is preferred due to increased freedom for the selection of supports. The added amount of white pigments to the resins in the interlayer is preferably 30 - 300 parts by weight in terms of solids of white pigments with respect to 100 parts by weight of resinous solids, but in order to enhance concealing properties, it is more preferable to use the white pigments in an amount ranging from 100 to 300 parts by weight.

Employed as interlayers may be ones which are formed by curing thermoplastic resins, thermally curable resins, or thermoplastic resins having a functional group, by employing various additives or techniques. Specifically, it is possible to use resins which are formed by curing polyvinyl alcohol, polyvinylpyrrolidone, polyester, chlorinated polypropylene, modified polyolefin, urethane resins, acryl resins, polycarbonate, ionomers, or resins which are prepared by curing pre-polymers having a monofunctional and/or multifunctional hydroxyl group employing isocyanate.

(Thermal Transfer Image Receptive Layer: Heat Transfer Image Receptive Layer (also called Image Receptive Layer))

An image receptive layer on one side of a support is comprising dye fixers and binder resins, as well as, if desired, various additives such as releasing agents.

Employed as binder resins may be those known in the art. It is preferable to use those which are readily dyed with dyes. Specifically, it is possible to use polyolefin resins such as polypropylene, halogenated resins such as polyvinyl

chloride or polyvinylidene chloride, vinyl based resins such as polyvinyl acetate or polyacrylic acid ester, polyester resins such as polyethylene terephthalate or polybutylene terephthalate, as well as polystyrene based resins, polyamide based resins, phenoxy resins, copolymers of olefins such as ethylene or propylene with other vinyl based monomers, polyurethane, polycarbonate, acryl resins, ionomers, cellulose derivatives or mixtures thereof. Of these, preferred are polyester based resins as well as vinyl based resins.

It is preferable to add releasing agents to the above image receptive layer to minimize thermal fusion with the dye layer. Employed as releasing agents may be phosphoric acid ester based plasticizers, fluorine based compounds, and silicone oil (including reaction curable type silicone). Of these, preferred is silicone oil. Employed as silicone oil are dimethylsilicone and various types of modified silicone. Specifically employed are amino-modified silicone, epoxy-modified silicone, alcohol-modified silicone, vinyl-modified silicone, and urethane-modified silicone. These may be blended and then employed. Further, these may be polymerized utilizing various reactions and then employed. Releasing agents may be employed individually or in combinations of at least two types. Further, the added amount of releasing agents is preferably 0.5 - 30 parts by weight with respect to 100 parts by weight of the image receptive layer forming resins. In the case in which the added amount is not within this range, problems occasionally occur such as fusion of a thermal transfer sheet with the image receptive layer of the image receptive sheet, or decrease in printing photo speed. Incidentally, these releasing agents are not added to the image receptive layer, but a releasing layer may be separately formed on the image receptive layer.

(Thermal Transfer Image Receptive Sheet: BC Layer, etc.)

Further, incorporated may be compounds for the purpose of enhancing mechanical tracking properties and antistatic properties, and providing writability and postal stamp adhesion properties. In order to achieve an antistatic function, formed may be a layer comprising electrically conductive resins such as acryl resins and electrically conductive fillers, and further a layer in which antistatic agents such as fatty acid ester, sulfuric acid ester, phosphoric acid ester, amides, quaternary ammonium salts, betaines, amino acids, or ethylene oxide addition products are added.

The amount of antistatic agents to be used varies depending on the layer to which antistatic agents are added, as well as the types thereof. In any case, however, it is preferable that the electric resistance value of the surface of image receptive sheets is to reach at most $10^{13}$ ohms/cm$^2$. In the case in which it is at least $10^{13}$ ohms/cm$^2$, image receptive sheets adhere to each other due to electrostatic adhesion, causing paper feeding problems. In terms of weight, the used amount is preferably 0.01 - 3.0 g/m$^2$. When the used amount of antistatic agents is less than 0.01 g/m$^2$, antistatic effects are not sufficiently exhibited, while when it exceeds 3.0 g/m$^2$, the excessive amount becomes uneconomical and tackiness problems occasionally occur. A writing layer may be arranged on the entire surface of the image receptive sheet or formed on only part of the surface.

Further, for the purpose of enhancing tracking properties, the addition of nylon resin particles as well as higher fatty acid salts is also effective. Listed as nylon resin particles are, for example, nylon 12 and nylon 6 particles. These nylon resin particles may be employed individually or in combinations of at least two types.

Employed as higher fatty acid salts may, for example, be calcium stearate, magnesium stearate, barium stearate, and zinc stearate. Employed as a writing layer forming means may be conventional prior art printing coating means. The dried weight of the writing layer is about 0.5 - about 20 g/m$^2$.

(Thermal Transfer Image Receptive Layer Support)

Supports play a role in securing an image receptive layer, while heat is applied to them during thermal transfer. As a result, it is preferable that the supports exhibit mechanical strength which results in no problem in an excessively heated state.

Materials for such supports are not particularly limited, and examples include, but are not limited to, condenser paper, glassine paper, parchment paper, highly sized paper, synthetic paper (polyolefin based and polystyrene based), wood free paper, art paper, coated paper, cast-coated paper, wall paper, lining paper, synthetic resin or emulsion impregnated paper, synthetic rubber latex impregnated paper, synthetic resin internal paper, cardboard, cellulose fiber paper, as well as films comprising polyester, polyacrylate, polycarbonate, polyurethane, polyimide, polyether imide, cellulose derivatives, polyethylene, ethylene-vinyl acetate copolymers, polypropylene, polystyrene, acryl, polyvinyl chloride, polyvinylidene chloride, polyvinyl alcohol, polyvinyl butyral, nylon, polyether ether ketone, polysulfone, polyether sulfone, tetrafluoroethylene, perfluoroalkyl vinyl ether, polyvinyl fluoride, tetrafluoroethylene-ethylene, tetrafluoroethylene-hexafluoropropylene, polychlorotrifluoroethylene, and polyvinylidene fluoride. Further, it is possible to use white opaque film prepared by casting a mixture of these synthetic resins with white pigments and fillers or foamed sheets prepared by foaming these resins.

Further employed may be laminations which are prepared by optionally combining the above supports. Listed as examples of representative laminations include synthetic paper with cellulose fiber paper, or synthetic paper of

cellulose synthetic paper with plastic film. The thickness of these supports is optional and is commonly about 10 - about 300 μm.

In order to achieve higher photographic printing speed and to obtain higher image quality with even density as well as no white spots, it is preferable to arrange a layer having minute voids. Employed as layers having such minute voids are plastic film and synthetic paper having minute voids in the interior. Further, it is possible to form a layer having minute voids employing various coating systems. Preferred as plastic film or synthetic paper having minute voids are those prepared in such a manner that polypropylene as a primary component is blended with inorganic pigments and/or polymers incompatible with polypropylene, which are employed as a void forming initiator, and the resulting mixture is oriented in a film. In cases in which polyester is employed as a primary component, the resulting cushioning properties and adiabatic properties are inferior to those prepared by employing polypropylene as a primary component due to viscoelastic and thermal properties, whereby photographic printing speed is lower and uneven density tends to occur.

In terms of these aspects, the elastic modulus of plastic film and synthetic paper at 20 °C is preferably 5 x $10^8$ - 1 x $10^{10}$ Pa. Since these plastic film and synthetic paper are commonly filmed by biaxial orientation, they are subject to contract by heating. In cases in which these are allowed to stand at 110 °C for 60 minutes, the coefficient of contraction is 0.5 - 2.5 percent. Each of the above plastic film and synthetic paper may form a single layer containing minute voids or may form a multilayer structure of a plurality of layers. In cases of a multilayer structure, all the layers may contain minute voids or there may be layer(s) which contain no minute voids. If desired, white pigments may be incorporated in these plastic films and synthetic paper as a concealing agent. In order to enhance whiteness, incorporated may be optical brightening agents. The thickness of layers containing minute voids is preferably 30 - 80 μm.

It is possible, employing an appropriate coating method, to form a layer having minute voids on a support. Employed as plastic resins may be prior art resins such as polyester, urethane resins, polycarbonate, acryl resins, polyvinyl chloride, or polyvinyl acetate. They may be employed individually or in combinations of a plurality of types. Employed as supports may be the above-mentioned various types of paper, synthetic paper, unwoven fabric, and plastic films.

Further, to minimize curling, if desired, it is possible to arrange, on the surface on the side opposite the side on which an image receptive layer is arranged, a layer comprising resins such as polyvinyl alcohol, polyvinylidene chloride, polyethylene, polypropylene, modified olefin, polyethylene terephthalate, or polycarbonate, or synthetic paper. Employed as lamination methods may be prior art methods such as a dry lamination method, a non-solvent (hot melt) lamination method, or an EC lamination method. Of these, preferred are the dry lamination method and the non-solvent lamination method. Listed as a suitable adhesive for the non-solvent lamination method is, for example, Takenate 720L produced by Takeda Chemical Industries, Ltd., while listed as suitable adhesives for the dry lamination method are Takeluck A969/Takenate A-5 (3/1), produced by Takeda Chemical Industries, Ltd., and Polysol PSA SE-1400 and Vinylol PSA AV-6200 Series, both produced by Showa Highpolymer Co., Ltd. The used amount of these adhesives is commonly in the range of about 1 - about 8 g/$m^2$ in terms of solids, but is preferably in the range of 2 - 6 g/$m^2$.

When a plastic film and a synthetic paper, each of these, or one of the various type of paper and a plastic film or synthetic paper, as described above, are laminated, it is possible to adhere them via an adhesion layer.

With the aim of enhancing adhesion between the above-mentioned support and thermal transfer image receptive layer, it is preferable that the support surface is subjected to various types of primer treatments and corona discharge treatment.

(Layer Structures and Coating Methods)

It is possible to form the above thermal transfer image receptive layer as follows. Applied onto a support is a liquid coating composition, which is prepared by dissolving or dispersing necessary components in water or solvents such as organic solvents, employing a common method such as a bar coater, a gravure printing method, a screen printing method, a roller coating method, a reverse roller coating method using a gravure plate, a curtain coating method, or an extrusion method, and subsequently dried. Other layers are also formed in the same manners as for the above image receptive layer. Further, other than the method in which, as noted above, the thermal transfer image receptive layer is formed by applying the liquid coating composition directly onto a support and subsequently dried, an image receptive layer is previously formed on another support and the resulting image receptive layer is transferred onto a support. Still further, it is possible to simultaneously coat at least two layers or to simultaneously coat all the layers.

The thickness of the image receptive layer after drying is preferably about 0.1 - 10 μm.

(Recording Methods)

One example of the thermal transfer recording method of the present invention will now be explained.

An embodiment will be described with reference to the drawing in the case in which a protective layer region

is fed in the surface order with the ink layer of an ink sheet.

Fig. 1(a) and Fig. 1(b) are views each showing an ink sheet which is one of the embodiments of the present invention. In Fig. 1(a), yellow (2Y), magenta (2M), and cyan (2C) ink layers 2 are arranged in the face order on the same plane as support 3, and the protective layer region is arranged between 2C and 2Y. Incidentally, in Fig. 1(a), no space between each of the layers is arranged, but a space may appropriately be arranged to match with the control method of a thermal transfer recording apparatus. Further, in order to accurately cue up each layer, it is preferable to arrange a detection mark on the ink sheet, but such arranging methods are not particularly limited.

In Fig. 1(a), the ink layer and the protective region are arranged on the same plane of the substrate. Obviously, however, as shown in Fig. 1(b), each of the layers may be arranged on separate supports (3 and 3').

In regard to the definition of ink layers, in the case in which reactive dyes are employed, dyes themselves incorporated in the ink layer are compounds prior to reaction. Therefore, in terms of precise definition, they may not be designated as Y, M, and C dyes. However, since they finally form Y, M, and C images, herein, they are designated as above.

(Chelate Recording Apparatus)

Employed as a thermal transfer recording apparatus employed in the present invention may, for example, be the apparatus shown in Figs. 2(a), 2(b) and 2(c). In Figs. 2(a), 2(b) and 2(c), numeral 10 is an ink sheet feeding roller, 15 is an ink sheet, 11 is a winding roller which winds used ink sheet 15, 12 is a thermal head, 13 is a platen roller, and 14 is an image receptive sheet inserted between thermal head 12 and platen roller 13.

When an image is formed employing an ink sheet such as that shown in Fig. 1(a), while employing the thermal transfer recording apparatus shown in Fig. 2(a), initially, yellow dye containing region 2Y of the ink sheet is placed face-to-face with the image receptive layer of an image receptive sheet, and yellow dyes in the yellow layer of the aforesaid region are heated employing a thermal head based on image data and transferred to the image receptive layer to form a yellow image. Subsequently, in the same manner as above, magenta dyes are transferred imagewise onto the resulting yellow image from the ink layer of magenta dye containing region 2M, and subsequently, in the same manner as above, cyan dyes are transferred imagewise onto the resulting transferred image form the ink layer of cyan dye containing region 2C. Finally, a protective layer is transferred onto the entire surface of the resulting image from protective layer region 1, whereby the formation of an image is completed.

In Fig. 2(b), an embodiment utilizing Fig. 1(b) is shown. In this case, in a dye transfer apparatus, initially, yellow dye containing region 2Y of an ink sheet is placed face-to-face with the image receptive layer of an image receptive sheet, and yellow dyes in the ink layer of the aforesaid region is heated based on image data employing a thermal head, and then transferred onto an image receptive layer to form a yellow image. Subsequently, in the same manner as above, magenta dyes are transferred imagewise onto the resulting yellow image from the ink layer of magenta dye containing region 2M, and subsequently, in the same manner as above, cyan dyes are transferred imagewise onto the transferred image from the ink layer of cyan dye containing region 2C to form a dye transferred image. Finally, by employing a protective layer transferring apparatus, a protective layer is transferred onto the surface of the entire image from protective layer region 1, whereby the formation of an image is completed.

Shown in Fig. 2(c) is a case in which each of the color ink sheets is individually used and recording is conducted employing an apparatus corresponding to each of them. In this case, initially, dyes on the yellow ink sheet are transferred onto an image receptive sheet based on image data, employing the Y transfer apparatus to form a yellow image. Subsequently, dyes on the magenta ink sheet are transferred imagewise in the same manner as above onto the yellow image, employing the magenta transfer apparatus, and dyes on the cyan ink sheet are then transferred onto the above (Y + M) image based on image data, employing the cyan transfer apparatus. Finally, in the same manner as in the case of Fig. 2(b), a protective layer is transferred on the entire surface of the resulting image from protective layer region 1, employing the protective layer transfer apparatus, whereby the formation of an image is completed.

In the thermal transfer printer according to the present invention, it is preferable to make it possible to select the production of a glossy surface or a matte surface in one printer, because it is possible to produce desired surface printed matter employing only one printer. The selection methods are not particularly limited. For example, stored in a thermal transfer recording apparatus are control data corresponding to a glossy surface and a matte surface of the present invention, and control data which is easily selected by an operator are read, whereby a control section may be controlled. In cases in which a printer is connected to a personal computer, the control data is stored in the personal computer and the control data which is easily selected by an operator may be transmitted to a printer. Further, in cases in which heating is performed employing a heating roller, it is possible to produce different surface recorded materials in such a manner that surface modifying materials such as a releasing sheet which results in glossiness or a surface-roughened sheet which results in a matte surface is fed onto the surface of an image receptive layer and the sheet is heated from the reverse side employing a heating roller.

(Post-Heating Methods)

In the present invention, recorded materials produced by thermal transfer recording may be subjected to a heating process after image formation.

Heating after image formation is conducted with the aim of fixing of transferred dyes in the image receptive layer.

Listed as heating methods are: employing a thermal head commonly used in thermal transfer recording, a method employing a heating roller, employing a heater or a hot air blower, and employing electron beam radiation or infrared radiation.

Either one side or both sides of recording materials can be heated, or both sides may be simultaneously heated.

Employed as a preferred heating method in the present invention is either the method employing thermal heads or the method employing heating rollers.

## EXAMPLES

The present invention will now be described with reference to examples. Parts and percent in the description are based on weight unless otherwise specified.

<Preparation of Ink Sheet>

A support for ink sheets was prepared as follows. A 0.5 µm thick primer layer comprising urethane based resins was provided on one side of a 6 µm thick PET film (K-203E-6F, produced by Diafoil Hoechst Co., Ltd.), while a 1 µm thick silicone resin layer was provided on the other side (the reverse surface) as a heat resistant lubricating layer.

<Preparation of Ink Sheet 1>

Ink Sheet 1 was prepared in the following manner. On the ink sheet support formed were, in the face order, each of the yellow (Y), magenta (M), and cyan (C) dye layers, composed as described below as well as a transparent protective layer laminated by three layers consisting of a releasing layer, a transferable transparent protective layer, and an adhesion layer. The releasing layer was formed to result, after drying, in a coated weight of 0.4 g/m$^2$, employing a gravure coating method, and the transferable transparent protective layer was formed to result, after drying, in a coated weight of 2.0 g/m$^2$, employing a gravure coating method, while the adhesion layer was formed to result, after drying, in a coated weight of 1.0 g/m$^2$ employing a gravure coating method.

| (Y Dye Layer Liquid Coating Composition) | |
|---|---|
| Post-chelate dye: Exemplified Compound (1)-32 | 1.0 part |
| Polyvinyl butyral (KY-24, produced by Denki Kagaku Kogyo Co., Ltd.) | 5.5 parts |
| Urethane modified silicone resin (DAIAROMA SP-2105, produced by Dainichiseika Color & Chemicals Mfg. Co., Ltd.) | 1.5 parts |
| Methyl ethyl ketone | 80.0 parts |
| Butyl acetate | 10.0 parts |

| (M Dye Layer Liquid Coating Composition) | |
|---|---|
| Post-chelate dye: Exemplified Compound (2)-38 | 1.0 part |
| Polyvinyl butyral (KY-24, produced by Denki Kagaku Kogyo Co., Ltd.) | 5.5 parts |
| Urethane modified silicone resin (DAIAROMA SP-2105, produced by Dainichiseika Color & Chemicals Mfg. Co., Ltd.) | 1.5 parts |
| Methyl ethyl ketone | 80.0 parts |
| Butyl acetate | 10.0 parts |

| (C Dye Layer Liquid Coating Composition) | |
|---|---|
| Post-chelate dye: Exemplified Compound (4)-27 | 1.0 part |
| Polyvinyl butyral (KY-24, produced by Denki Kagaku Kogyo Co., Ltd.) | 5.5 parts |
| Urethane modified silicone resin (DAIAROMA SP-2105, produced by Dainichiseika Color & Chemicals Mfg. Co., Ltd.) | 1.5 parts |
| Methyl ethyl ketone | 80.0 parts |
| Butyl acetate | 10.0 parts |

| (Releasing Layer Liquid Coating Composition) | |
|---|---|
| Minute inorganic particles (Colloidal Silica, produced by Nissan Chemical Industries, Ltd.) | 10.0 parts |
| Polyvinyl alcohol (manufactured by Nippon Synthetic Chemical Industry Co., Ltd.) | 8.0 parts |
| Water | 50.0 parts |
| Ethanol | 40.0 parts |

| (Transferable Transparent Protective Layer Liquid Coating Composition) | |
|---|---|
| Vinyl chloride/vinyl acetate copolymer (#1000A, produced by Denki Kagaku Kogyo Co., Ltd.) | 15.0 parts |
| Copolymer resin combined with a reactive ultraviolet radiation absorbing agent via reaction (UVA635L, produced by BASF Japan) | 20.0 parts |
| Methyl ethyl ketone | 50.0 parts |
| Toluene | 50.0 parts |

| (Adhesion Layer Liquid Coating Composition) | |
|---|---|
| Vinyl chloride/vinyl acetate copolymer (#1000A, produced by Denki Kagaku Kogyo Co., Ltd.) | 20.0 parts |
| Microsilica | 1.0 part |
| Methyl ethyl ketone | 40.0 parts |
| Toluene | 40.0 parts |

<Preparation of Thermal Transfer Image Receptive Sheet> (Support)

Porous PET Sheet A was prepared as follows. The first layer, the second layer, and the third lawyer, described below, were simultaneously extruded at 300 °C employing an extruder, whereby a sheet comprising the three layers was formed on a stainless steel belt, and subsequently, the resulting sheet was cooled and solidified. The resulting film was stretched three times in the conveying direction while conveyed by rollers heated at 115 °C, and subsequently, the resulting film was stretched three times at right angles to the conveying direction, while holding both ends with clips. Thereafter, the stretched film was subjected to thermal fixing at 200 °C and cooled to room temperature, whereby a film shaped Porous PET Sheet A at 0.7 specific gravity with a first layer thickness of 8 µm, a second lawyer thickness of 35 µm, and a third layer thickness of 8 µm was prepared.

(Composition of Porous PET Sheet A)

| Composition of First Layer and Third Layer PET | |
|---|---|
| Composition of Second Layer PET | 90 parts |
| PET-PTMG (which was produced by adding PTMG (polytetramethylene glycol of a molecular weight of 4,000) so that during polymerization, the ratio of PET to PTMG was 1 : 1) | 1 part |
| Syndiotactic styrene (ZAREC S10, manufactured by Idemitsu Petrochemical Co., Ltd.) | 6 parts |

Both sides of the PET sheet prepared as above were subjected to a corona discharge treatment. Subsequently, one side of a 110 μm thick woodfree paper at a basic weight of 130 g/m$^2$ was allowed to adhere to Porous PET Sheet A, employing Polysol PSA SE-1400 (produced by Showa Polymer Co., Ltd.) as an adhesive, by passage through a laminator at 140 °C. Further, low density polyethylene at a density of 0.92 containing anatase type titanium oxide in an amount of 9.5 percent by weight was extruded onto the other side to result in a thickness of 40 μm, employing a melt extrusion coating method, whereby Thermal Transfer Image Receptive Layer Support 1 was prepared.

<Preparation of Thermal Transfer Image Receptive Layer 1 (Comparative Example)>

The sublayer composed as described below was applied onto the surface of the porous PET sheet of thermal transfer image receptive sheet support, and the coating was dried at 120 °C for one minute.

Subsequently, the image receptive layer composed as described below was applied on the above coating to result, after drying, in a coated weight of 2.5 g/m$^2$, and the resulting coating was dried at 130 °C for two minutes. Thereafter, the resulting film was slit to a width of 152 mm, whereby rolls of Thermal Transfer Image Receptive Sheet 1 were prepared.

| (Sublayer Forming Liquid Coating Composition) | |
|---|---|
| Acryl based emulsion (a 35 percent aqueous solution of NICASOL A-08, produced by Nippon Carbide Industries Co., Ltd.) | 5.7 parts |
| Pure water | 94.0 parts |

| (Formulation of Image Receptive Layer Liquid Coating Composition) | |
|---|---|
| Vinyl chloride/vinyl acetate copolymer resin (#1000GK, manufactured by Denki Kagaku Co., Ltd.) | 42.0 parts |
| Metal Source 1 | 18.0 parts |
| Epoxy modified silicone (KF-393, produced by Shin-Etsu Chemical Co., Ltd.) | 0.7 part |
| Amino modified silicone (KS-393, produced by Shin-Etsu Chemical Co., Ltd.) | 0.3 part |
| Methyl ethyl ketone | 20.0 parts |
| Toluene | 20.0 parts |
| Metal Source 1 (MS-1) : $Ni^{2+}[C_7H_{15}COC(COOCH_3)=C(CH_3)O-]_3$, molecular weight: 541.30 | |

<Preparation of Thermal Transfer Image Receptive Sheet 2 - 11>

Thermal Transfer Image Receptive Sheets 2 - 11 were prepared in which Metal Source 1 in Thermal Transfer Image Receptive Sheet 1 was varied as listed in Table 4. The amount of each of the metal sources to be coated was added so that the amount of metal ions to be contained was the same depending on the molecular weight of the each of the metal sources.

Table 4

| Thermal Transfer Image Receptive Sheet | Compound Represented by Formula (I) | Value of of logP | Metal Source | $M^{2+}$ | Molecular Weight | Relative Value/MS-1 | |
|---|---|---|---|---|---|---|---|
| 1 | - | 2.72 | MS-1 | $Ni^{2+}$ | 541.3 | 1.00 | Comp. |
| 2 | I-1 | 3.77 | II-1 | $Ni^{2+}$ | 725.49 | 1.34 | Inv. |
| 3 | I-1 | 3.77 | III-1 | $Cu^{2+}$ | 730.35 | 1.35 | Inv. |
| 4 | I-8 | 6.28 | II-8 | $Ni^{2+}$ | 865.75 | 1.60 | Inv. |
| 5 | I-8 | 6.28 | III-8 | $Cu^{2+}$ | 870.61 | 1.61 | Inv. |
| 6 | I-19 | 7.08 | II-19 | $Ni^{2+}$ | 921.85 | 1.70 | Inv. |
| 7 | I-19 | 7.08 | III-19 | $Cu^{2+}$ | 926.71 | 1.71 | Inv. |
| 8 | I-22 | 6.81 | II-22 | $Ni^{2+}$ | 961.83 | 1.78 | Inv. |

Table 4 (continued)

| Thermal Transfer Image Receptive Sheet | Compound Represented by Formula (I) | Value of of logP | Metal Source | $M^{2+}$ | Molecular Weight | Relative Value/MS-1 | |
|---|---|---|---|---|---|---|---|
| 9 | - | 1.58 | MS-2 | $Ni^{2+}$ | 585.23 | 1.08 | Comp. |
| 10 | - | 6.94 | MS-3 | $Ni^{2+}$ | 893.81 | 1.65 | Comp. |
| 11 | - | 5.34 | MS-4 | $Ni^{2+}$ | 893.81 | 1.65 | Comp. |
| Comp.: Comparative Example Inv.: Present Invention | | | | | | | |

Example 1

Each of the image receptive layer portions of Thermal Transfer Image Receptive Sheets 1 - 11 was placed face-to-face with the dye layer portion of Ink Sheet 1 and placed in an apparatus loaded with a thermal head which was a 300 dpi (which represents the number of dots per inch, namely 2.54 cm) line head in which the shape of the resistor was a square. Each of the faced ones was brought into pressure contact with the thermal head and platen roller. During pressure contact, heat was applied to the dye layer and a yellow, magenta, cyan, and neutral (in which the aforesaid three colors overlapped) step pattern in which applied energy was successively increased with in the range of 5 - 80 mJ/$mm^2$ and subsequently a transparent protective layer was conveyed at a conveying rate of 10 m/second and a conveying length of 85 µm per line, whereby Samples 1-(1) through 1-(11) in which dyes were transferred to the image receptive layer were prepared.

(Light-fastness, Moisture Resistance, Heat Resistance, Yellowing, and Image Bleeding)

After density Ci of each of Samples 1-(1) through 1-(11), after transfer, was determined employing X-rite 310, Samples were irradiated with xenon light at 85,000 lux over a period of 14 days, employing a weather meter produced by Atlas, and thereafter density Cf was again determined, whereby residual dye ratio, (Cf/Ci) x 100 was obtained.

Maximum density (Dmax) of yellow, magenta, cyan, and neutral was listed. Further, with regard to each density of yellow, magenta, cyan, and neutral, when the residual dye ratio was at least 90 percent, light-fastness was ranked as A, when it was at least 80 percent, light-fastness was ranked as B, while when it was at least 70 percent, light-fastness was ranked as C.

Further, Samples 1-(1) through 1-(11) were allowed to stand under high temperature and high humidity conditions of 60 °C and 90 percent relative humidity, as well as a high temperature condition of 77 °C and 10 percent relative humidity over a period 14 days, and any change of white background and chromatic bleeding were visually evaluated. White background change: A: no yellowing was noted, B: yellowing was noted

Chromatic bleeding: A: no bleeding was noted, B: bleeding was noted

Table 5 shows the results.

Table 5

| Sample No. | Thermal Transfer Image Receptive Sheet | Maximum Density | | | Lightfastness | | | | 60 °C 90% RH | | 77 °C 10% RH | | Remarks |
|---|---|---|---|---|---|---|---|---|---|---|---|---|---|
| | | Y | M | C | Y | M | C | Bk | White Background Change | Chromatic Bleeding | White Background Change | Chromatic Bleeding | |
| 1-(1) | 1 | 1.85 | 2.08 | 2.13 | A | B | C | B | B | B | B | B | Comp. |
| 1-(2) | 2 | 2.25 | 2.18 | 2.21 | A | A | B | B | A | A | A | A | Inv. |
| 1-(3) | 3 | 2.28 | 2.15 | 2.29 | A | B | B | B | A | A | A | A | Inv. |
| 1-(4) | 4 | 2.38 | 2.33 | 2.4 | A | A | A | A | A | A | A | A | Inv. |
| 1-(5) | 5 | 2.34 | 2.3 | 2.38 | A | A | B | B | A | A | A | A | Inv. |
| 1-(6) | 6 | 2.41 | 2.35 | 2.44 | A | A | A | A | A | A | A | A | Inv. |
| 1-(7) | 7 | 2.33 | 2.28 | 2.25 | A | A | B | B | A | A | A | A | Inv. |
| 1-(8) | 8 | 2.25 | 2.28 | 2.23 | A | B | A | A | A | A | A | A | Inv. |
| 1-(9) | 9 | 2.15 | 2.18 | 2.14 | B | C | C | C | A | A | B | A | Comp. |
| 1-(10) | 10 | 2.19 | 2.21 | 2.16 | B | C | C | C | A | A | B | A | Comp. |
| 1-(11) | 11 | 2.15 | 2.19 | 2.15 | B | B | C | C | A | A | B | A | Comp. |

Comp.: Comparative Example    Inv.: Present Invention

Based on the above, in cases in which the thermal transfer image receptive sheets of the present invention were employed, it was found that images were obtained which exhibited excellent storage stability, and particularly, stabilized images were obtained which resulted in no yellowing of white background under high temperature and high humidity conditions or under high temperature condition.

Example 2

Thermal Transfer Image Receptive Sheets 1-1 through 1-6 were prepared by varying the amount of Metal Source 1 to be added to the image receptive layer as listed in Table 6. Herein, the added amount is a relative value of Example 1. Further, Thermal Transfer Image Receptive Sheets 2, 5, 6, and 8 were prepared in the same manner by varying the amount of Metal Source in the image receptive layer. In this case, the added amount was the same as MS-1.

Table 6

| Previous Thermal Transfer Image Receptive Sheet No. | New Thermal Transfer Sheet No. | | | | | |
|---|---|---|---|---|---|---|
| | Added Amount of Metal Source | | | | | |
| | 0.6 | 0.8 | 1 | 1.2 | 1.4 | 1.6 |
| 1 | 1-1 | 1-2 | 1-3 | 1-4 | 1-5 | 1-6 |
| 2 | 2-1 | 2-2 | 2-3 | 2-4 | 2-5 | 2-6 |
| 4 | 4-1 | 4-2 | 4-3 | 4-4 | 4-5 | 4-6 |
| 6 | 6-1 | 6-2 | 6-3 | 6-4 | 6-5 | 6-6 |
| 8 | 8-1 | 8-2 | 8-3 | 8-4 | 8-5 | 8-6 |

Samples 2-(1)-1 through 2-(8)-6 were prepared in the same manner as Example 1 by transferring dyes to each of these thermal transfer image receptive sheets, employing a thermal head. The resulting samples were evaluated in the same manner as Example 1 for maximum transfer density, light-fastness, white background change, and chromatic bleeding. Table 7 shows the results.

Table 7

| Sample No. | Thermal Transfer Image Receptive Sheet No. | Maximum Density (Dmax) | | | Lightfastness | | | | 60 °C 90% RH | | 77 °C 10% RH | | Re-marks |
|---|---|---|---|---|---|---|---|---|---|---|---|---|---|
| | | Y | M | C | Y | M | C | Bk | White Back-ground Change | Chro-matic Bleed-ing | White Back-ground Change | Chro-matic Bleed-ing | |
| 2-(1)-1 | 1-1 | 1.46 | 1.78 | 1.81 | B | C | C | C | A | A | A | A | Comp. |
| 2-(1)-2 | 1-2 | 1.66 | 1.92 | 1.99 | A | C | C | C | A | B | B | B | Comp. |
| 2-(1)-3 | 1-3 | 1.85 | 2.08 | 2.13 | A | B | C | B | B | B | B | B | Comp. |
| 2-(1)-4 | 1-4 | 1.9 | 2.11 | 2.16 | A | B | C | B | B | B | B | B | Comp. |
| 2-(1)-5 | 1-5 | 1.91 | 2.1 | 2.15 | A | B | C | B | B | B | B | B | Comp. |
| 2-(1)-6 | 1-6 | 1.91 | 2.12 | 2.17 | A | B | B | B | B | B | B | B | Comp. |
| 2-(2)-1 | 2-1 | 2.11 | 2.09 | 2.11 | A | B | C | B | A | A | A | A | Inv. |
| 2-(2)-2 | 2-2 | 2.14 | 2.12 | 2.15 | A | B | B | B | A | A | A | A | Inv. |
| 2-(2)-3 | 2-3 | 2.18 | 2.15 | 2.18 | A | B | B | B | A | A | A | A | Inv. |
| 2-(2)-4 | 2-4 | 2.23 | 2.18 | 2.21 | A | A | B | B | A | A | A | A | Inv. |
| 2-(2)-5 | 2-5 | 2.27 | 2.21 | 2.23 | A | A | B | B | A | A | A | A | Inv. |
| 2-(2)-6 | 2-6 | 2.31 | 2.25 | 2.28 | A | A | B | A | A | A | A | A | Inv. |
| 2-(4)-1 | 4-1 | 2.33 | 2.31 | 2.35 | A | A | A | B | A | A | A | A | Inv. |
| 2-(4)-2 | 4-2 | 2.35 | 2.31 | 2.37 | A | A | A | A | A | A | A | A | Inv. |
| 2-(4)-3 | 4-3 | 2.37 | 2.33 | 2.39 | A | A | A | A | A | A | A | A | Inv. |
| 2-(4)-4 | 4-4 | 2.36 | 2.32 | 2.38 | A | A | A | A | A | A | A | A | Inv. |
| 2-(4)-5 | 4-5 | 2.37 | 2.33 | 2.4 | A | A | A | A | A | A | A | A | Inv. |
| 2-(4)-6 | 4-6 | 2.38 | 2.33 | 2.4 | A | A | A | A | A | A | A | A | Inv. |
| 2-(6)-1 | 6-1 | 2.33 | 2.31 | 2.36 | A | A | A | B | A | A | A | A | Inv. |
| 2-(6)-2 | 6-2 | 2.35 | 2.32 | 2.38 | A | A | A | A | A | A | A | A | Inv. |
| 2-(6)-3 | 6-3 | 2.38 | 2.34 | 2.39 | A | A | A | A | A | A | A | A | Inv. |
| 2-(6)-4 | 6-4 | 2.4 | 2.34 | 2.41 | A | A | A | A | A | A | A | A | Inv. |
| 2-(6)-5 | 6-5 | 2.41 | 2.35 | 2.43 | A | A | A | A | A | A | A | A | Inv. |
| 2-(6)-6 | 6-6 | 2.41 | 2.35 | 2.44 | A | A | A | A | A | A | A | A | Inv. |
| 2-(8)-1 | 8-1 | 2.15 | 2.1 | 2.13 | A | B | B | B | A | A | A | A | Inv. |
| 2-(8)-2 | 8-2 | 2.17 | 2.14 | 2.16 | A | B | B | B | A | A | A | A | Inv. |
| 2-(8)-3 | 8-3 | 2.21 | 2.16 | 2.19 | A | B | A | B | A | A | A | A | Inv. |
| 2-(8)-4 | 8-4 | 2.25 | 2.18 | 2.21 | A | B | A | A | A | A | A | A | Inv. |
| 2-(8)-5 | 8-5 | 2.27 | 2.19 | 2.23 | A | B | A | A | A | A | A | A | Inv. |
| 2-(8)-6 | 8-6 | 2.28 | 2.21 | 2.25 | A | B | A | A | A | A | A | B | Inv. |

Comp.: Comparative Example Inv.: Present Invention

Based on the above results, in cases in which the thermal transfer image receptive sheets of the present invention were employed, it was noted that even though the added amount of the metal source was decreased, high photographic speed was ensured, and images exhibiting excellent storage stability were obtained with no chromatic bleeding regardless of the added amount.

Example 3 (Addition of MS to Protective Layer)

Ink sheets were prepared in such a manner that in the case in which Thermal Transfer Image Receptive Sheets 1-1, 2-1, 5-1, 6-1, and 8-1 were employed, each of the metal sources was added to a transferable transparent protective layer so that the total added amount of the metal source reached 1.0 (the same amount as for Thermal Transfer Image Receptive Sheet 1 of Example 1) after transferring the protective layer.

By employing these ink sheets, Samples 3-(1)-1 through 3-(6)-1 were prepared in the same manner as Example 1 by transferring dyes onto each of Thermal Transfer Image Receptive Sheets 1-1, 2-1, 3-1, 5-1, and 6-1, employing a thermal head.

In addition, by employing the same method as for Example 2, Samples 3-(9)-1, 3-(10)-1, and 3-(11)-1 were prepared employing each of the ink sheets which were prepared in such a manner that each of the metal sources was added to the transparent protective layer of each of Thermal Transfer Image Receptive Sheets 9-1, 10-1, and 11-1, which was prepared by adding 0.6 the amount of the metal source to each of Thermal Transfer Image Receptive Sheets 9, 10, and 11.

These samples were evaluated in the same manner as Example 1 for maximum transfer density, light-fastness, white background change, and chromatic bleeding. Table 8 shows the results.

Table 8

| Sample No. | Thermal Transfer Image Receptive Sheet No. | Maximum Density (Dmax) | | | Lightfastness | | | | 60 °C 90% RH | | 77 °C 10% RH | | Re-marks |
|---|---|---|---|---|---|---|---|---|---|---|---|---|---|
| | | Y | M | C | Y | M | C | Bk | White Back-ground Change | Chro-matic Bleed-ing | White Back-ground Change | Chro-matic Bleed-ing | |
| 3-(1)-1 | 1-1 | 1.52 | 1.83 | 1.88 | B | B | C | C | A | A | B | B | Comp. |
| 3-(2)-1 | 2-1 | 2.3 | 2.25 | 2.27 | A | A | B | A | A | A | A | A | Inv. |
| 3-(4)-1 | 4-1 | 2.38 | 2.36 | 2.39 | A | A | A | A | A | A | A | A | Inv. |
| 3-(6)-1 | 6-1 | 2.42 | 2.37 | 2.45 | A | A | A | A | A | A | A | A | Inv. |
| 3-(8)-1 | 8-1 | 2.25 | 2.21 | 2.26 | A | B | A | A | A | A | A | A | Inv. |
| 3-(9)-1 | 9-1 | 2.12 | 2.15 | 2.11 | B | C | C | C | A | A | B | A | Comp. |
| 3-(10)-1 | 10-1 | 2.13 | 2.17 | 2.12 | B | C | C | C | A | A | B | A | Comp. |
| 3-(11)-1 | 11-1 | 2.17 | 2.21 | 2.19 | B | B | C | C | A | A | B | A | Comp. |

Comp.: Comparative Example Inv.: resent Invention

In cases in which the thermal transfer image receptive sheets, as well as the metal source-added ink sheets of the present invention are employed, it is possible to provide images exhibiting excellent image lasting quality as well as higher photographic speed without an increase in the added amount of metal sources due to the fact that chelating reaction is promoted by enclosing the dyes with the metal sources after thermal dye transfer.

Example 4

Ink sheets were prepared in such a manner that each of the post-chelate dyes in the ink sheets prepared in Example 1 was replaced with each of the metal-chelating dyes. The types of chelating dyes are listed in Table 9.

Further, thermal transfer image receptive sheets were prepared in the same manner as Example 1, except that the metal source was omitted from the composition of the thermal transfer image receptive sheet of Example 1.

Samples 10-1 through 10-9 were prepared in the same manner as Example 1, in such a manner that by employing ink sheets as well as thermal transfer image receptive sheets prepared as above, yellow, magenta, and cyan dyes were transferred employing a thermal head.

Samples prepared via the present invention as well as comparative samples were determined employing the methods described below, and various types of durability (light-fastness, heat resistance, and moisture resistance) of metal-chelated dye images were evaluated. Table 9 shows the results.

- Evaluation Methods -

(Light-fastness)

The resulting chelated dye transferred images were irradiated with xenon light at 85,000 lux over a period of 14 days, and light-fastness was evaluated based on the residual dye ratio (in percent), represented by $(D_1/D_0) \times 100$, wherein $D_0$ represents density prior to light irradiation, while $D_1$ represents density after light irradiation.

(Heat Resistance)

The resulting chelated dye transferred images were stored under conditions of 77 °C and 10 percent relative humidity over a period of 14 days, and heat resistance was evaluated based on the residual dye ratio (in percent), represented by $(D_2/D_0)$ x 100, wherein $D_0$ represents density prior to storage, while $D_2$ represents density after storage.

(Moisture Resistance)

The resulting chelated dye transferred images were stored under conditions of 60 °C and 90 percent relative humidity over a period of 14 days, and moisture resistance was evaluated based on the residual dye ratio (in percent), represented by $(D_3/D_0) \times 100$, wherein $D_0$ represents density prior to storage, while $D_3$ represents density after storage.

Table 9

| Sample No. | Chelate Dye | | | Lightfastness | | | Moisture Resistance | | | Heat Resistance | | | Re-marks |
|---|---|---|---|---|---|---|---|---|---|---|---|---|---|
| | Y | M | C | Y | M | C | Y | M | C | Y | M | C | |
| 10-1 | Y-1 | M-1 | C-1 | 91 | 85 | 80 | 92 | 82 | 81 | 93 | 83 | 79 | Comp. |
| 10-2 | (5)-9 | (6)-9 | (7)-9 | 97 | 94 | 91 | 96 | 94 | 95 | 98 | 97 | 96 | Inv. |
| 10-3 | (5)-10 | (6)-10 | (7)-10 | 98 | 95 | 92 | 98 | 97 | 98 | 99 | 98 | 99 | Inv. |
| 10-4 | (5)-11 | (6)-11 | (7)-11 | 96 | 93 | 91 | 97 | 95 | 96 | 97 | 98 | 95 | Inv. |
| 10-5 | (5)-9 | (6)-9 | (7)-10 | 98 | 94 | 90 | 95 | 96 | 92 | 96 | 98 | 96 | Inv. |
| 10-6 | (5)-9 | (5)-10 | (7)-9 | 96 | 94 | 92 | 97 | 94 | 95 | 98 | 97 | 97 | Inv. |
| 10-7 | (5)-10 | (6)-9 | (7)-9 | 97 | 96 | 92 | 95 | 96 | 97 | 99 | 96 | 97 | Inv. |
| 10-8 | (5)-10 | (6)-9 | (7)-10 | 96 | 94 | 91 | 98 | 95 | 97 | 97 | 97 | 95 | Inv. |
| 10-9 | (5)-11 | (6)-10 | (7)-11 | 97 | 93 | 91 | 96 | 94 | 96 | 96 | 95 | 96 | Inv. |

Comp.: Comparative Example  Inv.: Present Invention

**Comparative Chelate Dyes**

Y-1

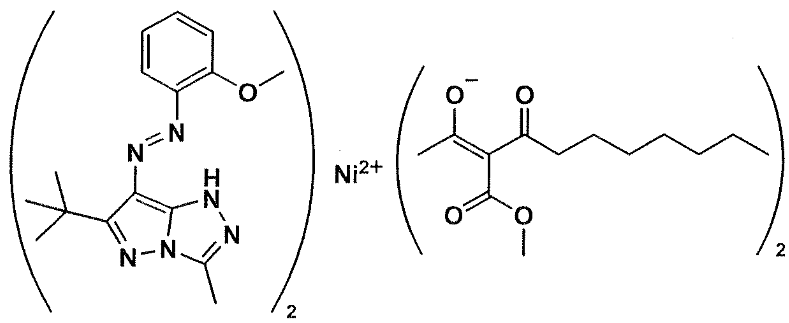

C-1

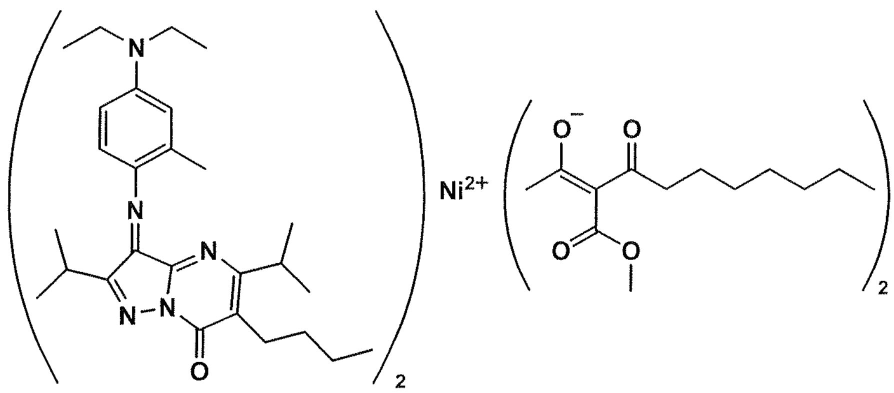

M-1

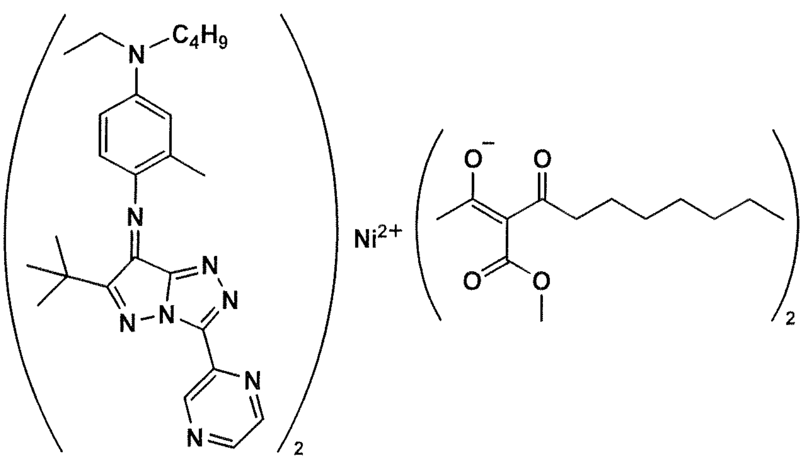

As be clearly seen from Table 9, the chelated dyes of the present invention resulted in excellent image durability in terms of light-fastness, heat resistance, and moisture resistance, compared to conventionally known metal-chelated dyes.

According to the present invention, it became possible to provide a thermal transfer recording material which results in high image quality (minimization of image bleeding), high photographic speed, as well as excellent storage stability, and image lasting quality (light-fastness), a thermal transfer image receptive sheet, and a thermal transfer image forming method capable of forming images exhibiting high quality as well as excellent image lasting quality. Surprisingly, in spite of the large molecular weight of incorporated metal sources, the photographic speed of the thermal transfer image receptive material of the present invention does not decrease even though its coated weight is the same as that of conventional known metal sources or is even decreased, and it is possible to attain excellent image lasting quality, whereby it is found that it is possible to achieve significant cost reduction. Further, in the thermal transfer material of the present invention, the addition of metal sources to the image receptive layer as well as the protective layer can increase its photographic speed. Still further, according to the present invention, it is possible to provide chelated dye images which exhibited excellent durability against light, heat and moisture.

## Claims

1. A thermal transfer recording material comprising a support having thereon a metal containing compound derived from a divalent metal salt and a compound represented by Formula (I):

Formula (I)

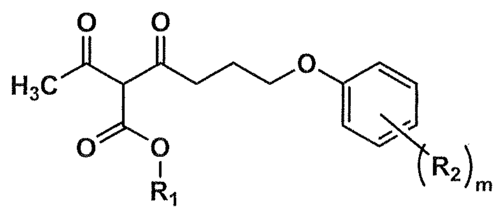

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provided that when m is 2 or more, a plurality of $R_2$s may be the same or different.

2. The thermal transfer recording material of claim 1,
wherein $R_1$ in Formula (I) is an alkyl group having two carbon atoms or more.

3. The thermal transfer recording material of claim 1,
wherein a hydrophilicity (a logP value) of the compound represented by Formula (I) is between 4.0 and 10.0.

4. A thermal transfer image receptive sheet comprising a support having thereon an image receptive layer containing the metal containing compound of claim 1.

5. A method of forming a metal-chelated dye image comprising the steps of:

(a) superimposing an ink sheet comprising a support having thereon an ink layer containing a metal-chelating dye onto the thermal transfer image receptive sheet of claim 4 so that a surface of the ink layer of the ink sheet faces a surface of the image receptive layer of the thermal transfer image receptive sheet; and
(b) imagewise heating the ink sheet and the thermal transfer image receptive sheet superimposed so as to form a metal-chelated dye by a reaction of the metal-chelating dye and the metal containing compound in the thermal transfer image receptive sheet of claim 4.

6. An ink sheet comprising a support having thereon an ink layer containing a metal-chelated dye derived from:

(a) a metal-containing compound derived from a divalent metal salt and a compound represented by Formula (I):

Formula (I)

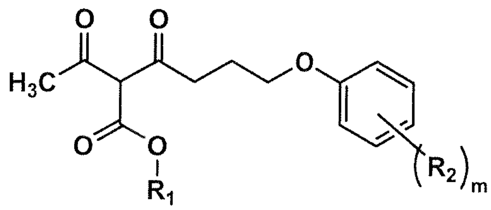

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provide that when m is 2 or more, a plurality of $R_2$s may be the same or different; and

(b) a metal-chelating dye represented by Formulas (1), (2) or (4):

Formula (1)

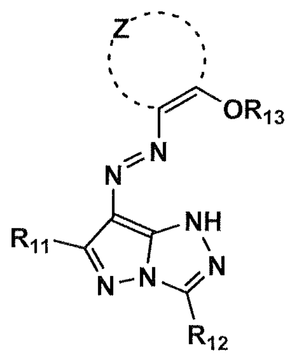

wherein $R_{11}$ and $R_{12}$ each represent a hydrogen atom or a substituent; $R_{13}$ represents an alkyl or an aryl group which may have a substituent; and Z represents a group of atoms necessary to form a 5- to 6-membered aromatic ring together with two carbon atoms;

Formula (2)

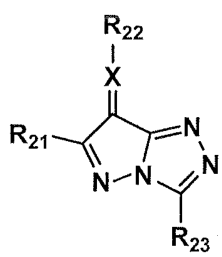

wherein $R_{21}$ represents a hydrogen atom, a halogen atom, or a univalent substituent; $R_{22}$ represents an aromatic carbon ring group or an aromatic heterocyclic group; X represents a methine group or a nitrogen atom; $R_{23}$ represents Formula (3), below; Y represents a group of atoms necessary to form a hetero-aromatic ring; and $X_1$ represents a carbon atom or a nitrogen atom;

Formula (3)

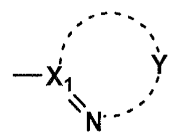

Formula (4)

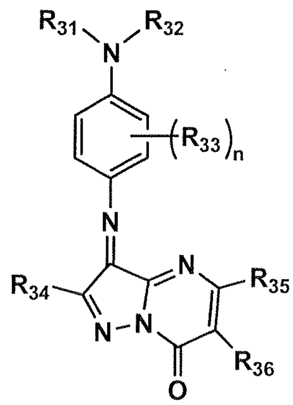

wherein $R_{31}$ and $R_{32}$ each represent a substituted or unsubstituted aliphatic group; $R_{33}$ represents a substituent; n represents an integer of 0 - 4, provided that when n is 2 or more, a plurality of $R_{33}$ may be the same or different; $R_{34}$, $R_{35}$, and $R_{36}$ each represent an alkyl group in which they may be the same or different, however, $R_{35}$ and $R_{36}$ each represent an alkyl group having 3 - 8 carbon atoms.

**7.** A method of forming a metal-chelated dye image comprising the steps of:

(a) superimposing the ink sheet of claim 6 onto a thermal transfer image receptive sheet comprising a support having thereon an image receptive layer including a metal containing compound so that a surface of the ink layer of the ink sheet faces a surface of the image receptive layer of the thermal transfer image receptive sheet; and
(b) imagewise heating the ink sheet and the thermal transfer image receptive sheet superimposed so as to form a metal-chelated dye by a reaction of the metal-chelating dye and the metal containing compound in the thermal transfer image receptive sheet.

**8.** A metal containing compound derived from a divalent metal salt and a compound represented by Formula (I):

Formula (I)

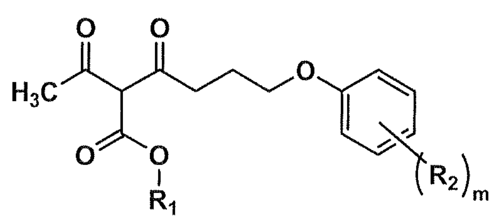

wherein $R_1$ represents an alkyl group of two carbon atoms or more, a cycloalkyl group, an alkenyl group, an alkynyl group, an aryl group, an aralkyl group or a heterocyclic group; $R_2$ represents a branched alkyl chain; and m represents an integer of 1 to 5, provide that when m is 2 or more, a plurality of $R_2$s may be the same or different.

**9.** Use of the metal containing compound of claim 8 for the manufacture of a thermal transfer recording material.

**10.** A thermal transfer recording material, comprising the compound of claim 8.

FIG. 1 ( a )

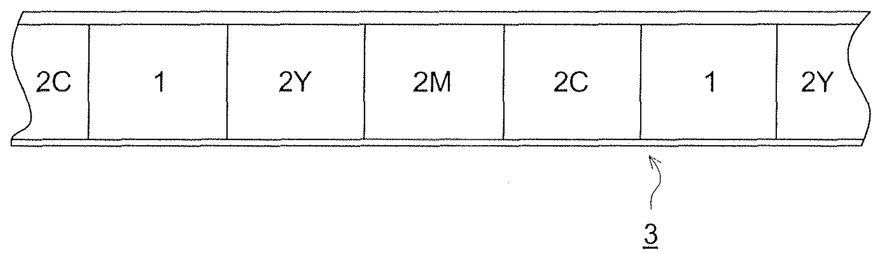


FIG. 1 ( b )

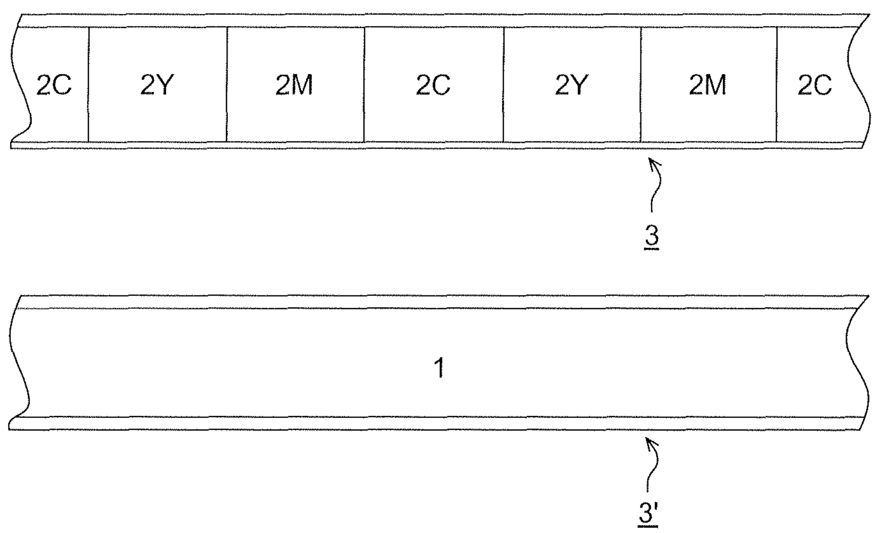

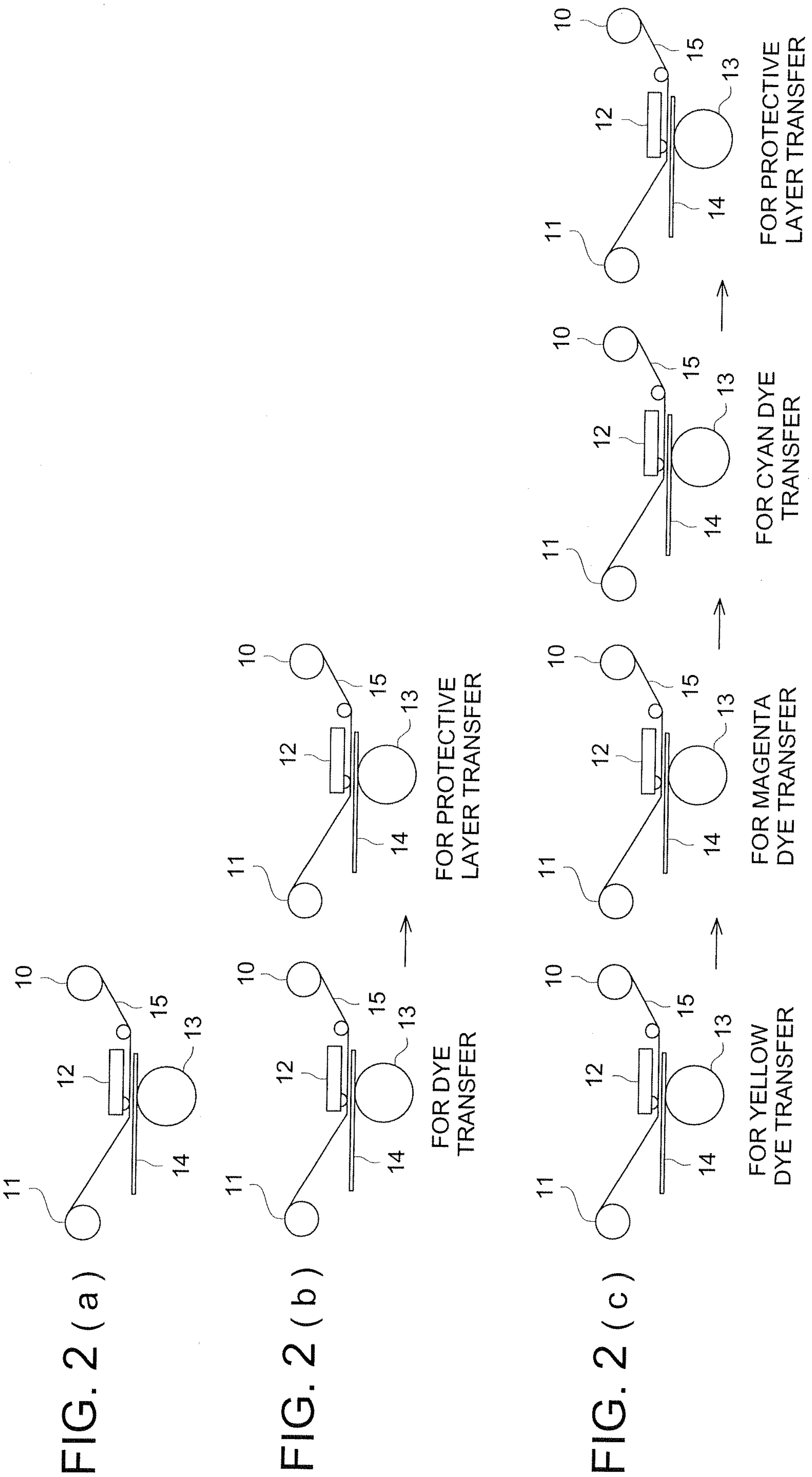


FIG. 2 (a)

FIG. 2 (b)

FIG. 2 (c)

European Patent Office

# EUROPEAN SEARCH REPORT

Application Number
EP 05 10 1218

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | JP 2003 238869 A (KONICA CORP) 27 August 2003 (2003-08-27) * page 5, compound I-4; paragraph (0029), (0047), page 17 * | 1-4,6, 8-10 | C07C69/738 B41M5/38 |
| A | EP 0 307 358 A (CIBA-GEIGY AG) 15 March 1989 (1989-03-15) * example 9; table 1 * | 8 | |

TECHNICAL FIELDS SEARCHED (Int.Cl.7)

C07C
B41M

The present search report has been drawn up for all claims

1

| Place of search | Date of completion of the search | Examiner |
|---|---|---|
| The Hague | 2 May 2005 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 10 1218

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2005

| Patent document cited in search report | Publication date | Patent family member(s) | Publication date |
|---|---|---|---|
| JP 2003238869 A | 27-08-2003 | NONE | |
| EP 0307358 A | 15-03-1989 | DE 3868371 D1 | 26-03-1992 |
| | | EP 0307358 A1 | 15-03-1989 |
| | | JP 1104027 A | 21-04-1989 |
| | | US 4992504 A | 12-02-1991 |

EPO FORM P0459

For more details about this annex : see Official Journal of the European Patent Office, No. 12/82